# EUROPEAN PATENT APPLICATION

(11) **EP 4 710 965 A1**
(43) Date of publication of application: **18.03.2026**
(21) Application number: 24199803.8
(22) Date of filing: 11.09.2024
(51) Int. Cl.: A61M 5/20, A61M 5/315, A61M 5/32, A61M 5/24

(54) **AUTOINJECTOR AND METHOD OF RECONSTITUTING AN AUTOINJECTOR**

(71) Applicant: medmix Switzerland AG, 9469 Haag (Rheintal) (CH)
(72) Inventor: Jones, Matthew Meredith, Warwick, CV34 4AB (GB); Morris, Anthony Paul, Warwick, CV34 4AB (GB); Martin, Jack Gerald, Warwick, CV34 4AB (GB)
(74) Representative: Manitz Finsterwald Patent- und Rechtsanwaltspartnerschaft mbB

(57) **Abstract**

The present invention relates to an autoinjector comprising a dual chamber component, a body, a spring chassis and a piston rod, wherein the piston rod is configured to be moved relative to the body and through the spring chassis, wherein the spring chassis has a first clip component and the piston rod has a second clip component configured complementary to the first clip component, wherein the first and second clip components are configured to engage once the piston rod has travelled a pre-determined distance through the spring chassis. The method further relates to a method of reconstituting a medicament stored in an autoinjector.

## Description

The present invention relates to an autoinjector comprising a dual chamber component, a body, a spring chassis and a piston rod, wherein the piston rod is configured to be moved relative to the body and through the spring chassis, wherein the spring chassis has a first clip component and the piston rod has a second clip component configured complementary to the first clip component, wherein the first and second clip components are configured to engage once the piston rod has travelled a pre-determined distance through the spring chassis. The method further relates to a method of reconstituting a medicament or mixing two or more medicaments stored in an autoinjector.

In various fields of applications such as for example the industrial, the medical and/or the dental sector it is common practice to provide dispensers or injectors that are capable of storing two or more components separately from one another and of mixing said components prior to said components being dispensed.

It has been found that this approach can also be favourable in the field of (self)application of certain medicaments by medical amateurs as certain medicaments consisting of two or more components can have an increased shelf life if stored separate from one another. Thereby the storage time for such medicaments can be enhanced drastically when the respective components are stored separately from one another such that they cannot interact with each other.

The crux with medical amateurs is that overcoming the bar for self-administering a medicament is often not easy. This can often lead to errors in application or even that sterility is broken which consequently then leads to even bigger issues.

This issue can be solved by providing (auto)injectors that are already prefilled with the right amount of each component of a certain medicament and that are configured to mix said components together before said mixture is then (self)injected by the user.

However, contrary to the dispensers used in the industrial sector for instance, it is necessary for (auto)injectors being used for medicaments to provide a sterile environment where the two or more components can be mixed with one another before the dispense of the mixed medicament as well as a sterile environment for the cannula with which said medicament is supposed to be injected.

In view of the above it is an object of the invention to provide an autoinjector with which all of the above issues can be overcome.

This object is satisfied by an autoinjector in accordance with the respective independent claim, such an autoinjector may comprise a dual chamber component, a body, a spring chassis and a piston rod, wherein the piston rod is configured to be moved relative to the body and through the spring chassis, wherein the spring chassis has a first clip component and the piston rod has a second clip component configured complementary to the first clip component, wherein the first and second clip components are configured to engage once the piston rod has travelled a pre-determined distance through the spring chassis.

In this connection it should be noted that a dual chamber component is a component having two separate chambers with a respective chamber being provided for a respective part of a medicament with each part having a longer storage life than the combined and mixed medicament.

By way of example such a dual chamber component can be a dual chamber cartridge comprising first and second stoppers separating the two chambers of the cartridge from one another and to which a needle assembly can be connected.

Such a dual chamber cartridge may comprise a proximal and a distal stopper that are arranged one after another along a longitudinal axis of the dual chamber cartridge.

In this connection the body comprises one or more housing parts that surround internal components of the autoinjector and to which one or more of the internal components, such as the drive chassis, a needle guard, and the dual chamber component.

The spring chassis is configured to be moved with respect to the body during a medicament dispensing process. It can thereby be driven by a spring acting thereon. The spring chassis is configured to entrain the piston rod such that the piston rod can engage one or more stoppers of the dual chamber component during the dispensing process respectively a dispensing operation.

During an activation process, also known as a medicament mixing or reconstitution process, the spring chassis is held in position with respect to the dual chamber component and the body, the piston rod is moved relative to the drive chassis such that it can act on the dual chamber component, for example the dual chamber cartridge, in order to bring about a mixing respectively a reconstitution process and to thereby activate the autoinjector for dispensing. If the medicament activation process is not carried out the piston rod can be held with respect to the body such that no dispense can take place.

The first and second clip components may be configured to cooperate with one another such that only following completion of the activation process a dispensing operation can take place. The pre-determined distance of travel of the piston rod through the spring chassis is selected such that the dual chamber component is acted on for a sufficient period that a medicament mixing process can take place.

Following engagement of the first and second clip components, the drive chassis may be configured to entrain the piston rod for a common axial movement of the drive chassis and the piston rod during the dispensing operation.

The drive chassis may also be configured in such a way that during the dispensing operation the drive chassis initially moves with respect to the piston rod until it comes into contact with a part thereof such that only after contact the two components move together.

The autoinjector may further comprise a sleeve in which a part of the body is received. Such a sleeve can be reliably used to carry out the activation process.

The piston rod may be in axial contact and/or held at the sleeve. In this way a movement of the sleeve can advantageously be transferred to a movement of the piston rod.

The piston rod may be releasably held at the sleeve. In this way the piston rod can move together with and also relative to the sleeve in different states of use of the autoinjector.

The piston rod may be configured to be disengaged from the sleeve once the first and second clip components have engaged. In this way the piston rod can be permitted to be moved relative to the sleeve.

The sleeve may be configured to move the piston rod on activation of the autoinjector. This initial movement of the piston rod can be used to bring about a mixing of medicament stored in the dual chamber component.

The sleeve may be configured to move the piston rod relative to the spring chassis during the activation of the autoinjector. This permits a construction size of the autoinjector to be minimized.

The piston rod may be configured to move relative to the first clip component of the spring chassis during the activation of the autoinjector. In this way a construction size of the autoinjector can be minimized.

The piston rod may comprise a tapered section adjacent to the second clip component. Such a tapered section may facilitate the relative movement between the piston rod and the drive chassis.

The tapered section may increase in size towards the second clip component. This further facilitates the relative movement between the two components.

The first clip component may be configured to move over the tapered section. In this way the first clip component is always stored close to the piston rod even during a storage state and in a activation state.

The body may be slideably mounted in the sleeve. In this way a relative sliding movement between the two components may effect a reconstitution process.

The autoinjector may further comprise a dial. Such a dial can be used to effect a reconstitution process by simply rotating the dial relative to the body.

The dial may be rotatable but axially fixed to the body. In such a way a construction size of the autoinjector can be minimized.

The piston rod may be releasably held at the dial. This, on the one hand, ensures a reconstitution process can take place and, on the other hand, can also ensure that the piston rod can be used for a dispensing process. The piston rod may be configured to be disengaged from the dial once the autoinjector has been activated.

The piston rod may be configured to be disengaged from the dial once the first and second clip components have engaged. In this way the piston rod can only be used for dispensing once the reconstitution process has taken place, as otherwise the piston rod will be held via the dial.

The dial may be configured to move the piston on activation of the autoinjector. In this way the dial can be used to carry out the reconstitution process.

The dial may be configured to rotate in order to drive the piston rod axially for the pre-determined distance. A rotational movement of the dial can thereby be beneficially used to entrain the piston rod.

The piston rod may be splined to a housing component and is only axially driven.

The piston rod may be splined to the dial and is axially and rotationally driven.

The piston rod may have an outer thread cooperating with an inner thread of the dial in order to axially and rotationally drive the piston rod proximally during the activation process, whereas for dispensing the piston rod does not rotate and is only axially driven by the piston rod.

The drive spring chassis may be an at least generally U-shaped drive spring chassis having first and second limbs, with the piston rod being guided in the first limb. Optionally a drive spring may be received in the second limb. Thus, the piston rod may move in parallel to a drive spring without a common axis.

The drive spring chassis may be slideably mounted in the body.

The autoinjector may further comprise a needle guard, wherein a distal movement of the needle guard relative to the body releases the spring chassis from the storage position to bring about, i.e. enable, a proximal movement of the spring chassis in the body for dispensing a medicament stored in said autoinjector in a dispensing operation.

The dual chamber component may comprise first and second chambers for first and second elements of a medicament that can each be stored in a respective one of the respective first and second chambers of the dual chamber component, with the first and second chambers being separated from one another by a proximal stopper in a storage state of the autoinjector.

The pre-determined distance of travel of the piston rod may be reached once a distal stopper of the dual chamber component has reached the proximal stopper. In this way one can reliably ensure a reconstitution process has taken place after a given distance of travel.

The autoinjector may further comprise said two elements of medicament and said second element stored in the chamber between the proximal and distal stopper, wherein a mixing operation of the autoinjector is completed once the second element has been mostly urged into the first chamber by a proximal movement of the distal stopper. Thereby a reconstitution process can be reliably carried out.

Said dispensing operation may only take place after the first and second clip components have engaged.

The spring chassis may be fixed to the body during a medicament mixing process.

The piston rod may move relative to the body during a medicament mixing process.

The piston rod and the spring chassis may move during a medicament dispensing operation.

The piston rod and the spring chassis may move relative to one another during a medicament mixing operation.

The second clip component of the piston rod may comprise a portion of reduced diameter with respect to directly adjacent sections of the piston rod. In this way the first clip component can move relative to parts of the second clip component prior to a dispensing operation.

The second clip component may comprise a proximal abutment arranged directly adjacent to the portion of reduced diameter. Such a proximal abutment can be reliably used to ensure that the piston rod can move together with the drive chassis during a dispensing operation.

During the dispensing operation, a movement of the piston rod may only take place after the first clip component engages the proximal abutment of the second clip component.

During the dispensing operation, the spring chassis may initially move relative to the body and to the piston rod prior to engagement of the first clip component with the proximal abutment.

The second clip component may further comprise a distal abutment.

The distal abutment and the proximal abutment may be arranged at oppositely disposed ends of the portion of reduced diameter. In this way an as compact as possible second clip component can be formed.

The first clip component can move relative to the portion of reduced diameter. Thereby a construction size of the autoinjector can be reduced.

According to a further aspect the present invention further relates to a method of reconstituting a medicament stored in an autoinjector, the method comprising the steps of:
moving a piston rod of an autoinjector through a drive chassis of said auto-injector to move a distal stopper of a dual chamber component proximally, e.g. into contact with a proximal stopper of the dual chamber component.

By means of such a method a reconstitution of medicament can be reliably ensured prior to a dispensing of medicament taking place.

According to a further aspect of the invention the autoinjector according to the invention comprises a dual chamber cartridge comprising two elements of a medicament that are each stored in a separate chamber of the dual chamber cartridge. Said dual chamber cartridge also comprises a bypass passage. The autoinjector further comprises at least one security mechanism configured to secure the autoinjector against premature mixing of the elements and dispensing of said mixed elements.

That is, according to the invention an autoinjector is provided that comprises a dual chamber cartridge for two elements of a medicament that is configured to store each element separately from one another. Furthermore, said dual chamber cartridge comprises a bypass passage that may be configured to enable the two elements to be mixed with one another when a mixing mechanism is triggered.

Furthermore, the autoinjector according to the invention comprises at least one security mechanism that is configured to secure the autoinjector against premature mixing of the elements and dispensing of said mixed elements. In this connection it should further be noted that the security mechanism according to the invention can further be configured to provide a sterile environment for the elements, and in particular a cannula of said autoinjector, prior to mixing the elements respectively dispensing said mixture, i.e. injecting the medicament into a patient. The security mechanism may additionally be configured to protect a user from being injured by the autoinjector by for example shielding a cannula from the environment when the autoinjector is in a storage state.

Hence, with the autoinjector according to the invention a medical amateur can easily mix and (self)apply a medicament that consists of two or more elements as the chambers of the autoinjector are prefilled with the right dosage of elements. Furthermore, by providing a security mechanism, the autoinjector according to the invention is safely secured against premature mixing and dispensing such that the storage time of the elements contained in the autoinjector can be enhanced as they cannot come in contact with one another before a mixing mechanism is triggered. Additionally, said security mechanism can be configured to provide a sterile environment for all parts of the autoinjector that are involved in the injection process of the mixed medicament.

According to a further aspect of the invention, which may in particular be combined with the first aspect of the invention, an autoinjector is provided comprising a dual chamber cartridge comprising two elements of a medicament that are each stored in a separate chamber of the dual chamber cartridge and the dual chamber cartridge comprising a bypass passage. The dual chamber cartridge further comprises a cap and seal assembly covering and fixed to an outlet from the dual chamber cartridge.

That is, according to this further aspect of the invention the autoinjector comprises a dual chamber cartridge comprising two chambers configured to store two elements of a medicament, i.e. one medicament element in each chamber. Furthermore, the autoinjector comprises a bypass passage that is configured to enable the two elements to be mixed with one another when a mixing mechanism is triggered by for example allowing one element to flow from its storage chamber into the other chamber.

Furthermore, the autoinjector according to the invention comprises a cap and seal assembly covering and fixed to an outlet from the dual chamber cartridge. Said cap and seal assembly is therefore configured to close an outlet of the cartridge such that the elements stored therein can be held in a sterile environment. Furthermore, the cap and seal assembly can be configured to prevent a premature mixing and dispensing of said elements.

In this connection it may be noted that the cap and seal assembly may comprise a cap and one or more seals surrounded by the cap. Hence, according to this embodiment the cap and seal assembly can be a two-step assembly that ensures safe sealing of the dual chamber cartridge at least as long as it is in its storage state.

According to another embodiment of the invention the dual chamber cartridge has an opening which is covered by the cap and sealed off by one of said one or more seals of the cap and seal assembly. That is, the one or more seals can for example be configured to directly close the opening of the dual chamber cartridge while the cap covering said one or more seals may be configured to not only cover the outlet of the dual chamber cartridge but also said seal. The cap may in particular further be configured to (completely) surround the opening as well as the one or more seals.

According to a further embodiment the cap and seal assembly comprises a first flange arrangement and the dual chamber cartridge comprises a second flange arrangement shaped complementary to the first flange arrangement and being engageable by the first flange arrangement. Hence, said first and second flange arrangements may enable a secure engagement between the cap and seal assembly and the dual chamber cartridge.

In this connection it should be noted that the first flange arrangement can surround said second flange arrangement. It may in particular be possible that said first flange arrangement completely surrounds said second flange arrangement such that the first flange arrangement can generally be put on said second flange arrangement to provide a safe connection between the two.

It may further be possible that the first flange arrangement is snap-fit into place at the second flange arrangement. Such a snap fit connection can ensure a safe connection between the first and the second flange arrangement. It may in particular even provide a releasable connection which can potentially be useful in case the autoinjector should be reusable or in case the different parts of the autoinjector are supposed to be discarded separately from one another.

According to another embodiment of the invention the autoinjector further comprises a cannula held in place at the dual chamber cartridge by the cap and seal assembly. Said cannula may for example be configured as an injection needle. That is, said cannula may extend from an outside of the dual chamber cartridge to an inside of the dual chamber cartridge by extending beyond said cap and/or said one or more seals of the cap and seal assembly.

In this connection it is noted that the cannula may be held in place at the dual chamber cartridge by one of said one or more seals of the cap and seal assembly. That is, said one or more seals can further be configured to hold said cannula in place.

It may further be possible that the cap and seal assembly further comprises a cannula shield covering the cap and seal assembly. Such a cannula shield can ensure safe handling of the autoinjector as it prevents a user from being unintentionally stuck by said cannula. Additionally, said cannula shield can ensure a sterile environment for the outlet of the autoinjector, respectively said cannula, before using it on a patient as the outlet respectively the cannula is protected from the environment.

According to a further embodiment of the invention the dual chamber cartridge further comprises a proximal and a distal stopper. Said stopper may for example be configured to seal the two chambers of the dual chamber cartridge at at least one end of each chamber. Additionally or alternatively they may be configured to push the elements stored in said respective chambers towards the outlet of the autoinjector upon mixing and/or dispensing of the elements.

According to another embodiment the dual chamber cartridge comprises a first chamber more proximally arranged than a second chamber, wherein the first chamber is filled with a solid material and the second chamber is filled with a liquid material. That is, each element of the medicament can be stored in a separate chamber until they are intnetionally mixed with one another in order to enhance the storage time of said elements.

In this connection it should be noted that the first chamber may be separated from the second chamber by the proximal stopper. According to this embodiment no further separation or chamber walls are necessary to separate one chamber from the other as the proximal stopper can act as such a separation wall.

It may further be possible that the proximal stopper is arranged distally of the bypass passage in a storage state of the autoinjector. This prevents the element stored in the second chamber from reaching the first chamber as the bypass passage is blocked by the proximal stopper.

In this connection it should further be noted that the proximal stopper can be arranged proximally of the bypass passage after a dispensing operation has taken place. That is, the proximal stopper may be arranged moveably in the autoinjector such that it may first be arranged distally of the bypass passage in a storage state and then proximally of the bypass passage after a dispensing operation has taken place.

By having the proximal stopper arranged proximally of the bypass passage after a dispensing operation has taken place it can further be prevented that any residuals of the medicament left in the first chamber can flow back into the second chamber behind the proximal stopper. This can be particularly important in applications where not all of the mixed medicament is injected at once but rather by two or more injections throughout a predetermined period of time.

According to a further embodiment the autoinjector further comprises a piston rod configured to act on said distal stopper and said proximal stopper and to thereby move the proximal stopper such that it is arranged at the bypass passage for urging the material into the proximal chamber past the proximal stopper via the bypass passage during a mixing process.

That is, according to this embodiment both the distal and the proximal stopper are arranged moveably in the dual chamber cartridge to be able to urge the elements that are arranged right in front of the respective stoppers towards the outlet of the dual chamber cartridge.

In this connection it should be noted that as the element that is stored in the distal chamber is urged through the bypass passage into the proximal chamber during the mixing process, it may be possible for the distal and the proximal stopper to come into contact to later move together as one.

It may thus be possible that the piston rod can be moved further for dispensing the medicament via the cap and seal assembly. That is, the piston rod can be moved such that it brings both stoppers towards the cap and seal assembly to urge the mixed material towards the outlet.

According to a further embodiment the one of said one or more seals of the cap and seal assembly sealing off the opening covers said opening. By sealing the opening it can for example be ensured that the mixed medicament can only be dispensed via the cap and seal assembly and/or a cannula.

According to yet another embodiment the autoinjector further comprises a cannula shield such as a rigid cannula shield covering a cannula of the dual chamber cartridge. Such a cannula shield can for example be provided to ensure safe handling of the autoinjector and to prevent injuries by shielding the user from the cannula. Furthermore, said cannula shield can be configured to provide a sterile environment for the cannula before the first use of the autoinjector.

According to a further aspect of the invention a method of mixing a medicament in an autoinjector is provided with the autoinjector comprising a dual chamber cartridge comprising first and second elements of a medicament, with the first and second elements each being stored in a separate chamber of the dual chamber cartridge, the dual chamber cartridge comprising a cap and seal assembly as well as a piston rod. The method according to the invention comprises the steps of: - moving the piston rod proximally towards a distal stopper and thereby moving said distal stopper towards a proximal stopper and reducing the distance between the distal stopper and said proximal stopper to thereby urge a first element of the medicament from a first chamber into a bypass passage to bypass the proximal stopper such that the first element comes into contact with the second element in the first chamber arranged between the cap and seal assembly and the proximal stopper.

That is, according to the invention it is possible to store two elements of a medicament separately in two different chambers in order to enhance the storage time of one or more of said elements.

Then, by moving the piston rod proximally towards the distal stopper such that said distal stopper is also guided towards the proximal stopper, the element that is stored in the distal chamber is urged towards said proximal stopper. When the pressure that acts on said proximal stopper is high enough, also the proximal stopper will start moving in a proximal direction thereby releasing the bypass channel such that the second element stored in the distal, i.e. the second, chamber can flow through said bypass channel to bypass the proximal stopper and to reach the proximal, i.e. the first, chamber where the first element is stored. This allows a mixing of the two elements.

It may additionally also be possible to move the piston rod further after the mixing of the elements is complete in order to urge the mixed medicament out of an outlet of the autoinjector in order to inject said medicament into a patient.

According to a further aspect of the invention an autoinjector comprising a dual chamber cartridge comprising two elements of a medicament that are each stored in a separate chamber of the dual chamber cartridge is provided. The dual chamber cartridge according to the invention comprises a bypass passage and a rubber septum and a cannula shield. The autoinjector further comprises a cannula carriage having a cannula with the cannula carriage being in contact with the dual chamber cartridge in a storage state of the autoinjector and the dual chamber cartridge being moveable towards the cannula carriage on activation of the autoinjector in order to penetrate the septum with the cannula.

That is, according to this further aspect of the invention the autoinjector comprises a dual chamber cartridge comprising two chambers configured to store two elements of a medicament, i.e. one element of medicament in each chamber.

Furthermore, the autoinjector comprises a bypass passage that may be configured to enable the two elements to be mixed with one another when a mixing mechanism and/or an activation mechanism is triggered.

According to this aspect of the invention the autoinjector further comprises a cannula carriage having a cannula with the cannula carriage being in contact with the dual chamber cartridge in a storage state. The cannula carriage may thus enable a safe connection of the cannula at the dual chamber cartridge.

However, in a storage state, said cannula carriage is only in contact with the dual chamber cartridge but the cannula itself does not yet extend into said dual chamber cartridge. This is only the case when the autoinjector is activated by moving the dual chamber cartridge towards the cannula carriage (or the other way around) such that the cannula can penetrate the septum of the dual chamber cartridge to extend beyond said septum into said dual chamber cartridge thereby providing a flow channel for the elements stored inside the autoinjector.

According to an embodiment of the invention a distal end of the cannula carriage is sealed off by a seal to make sure that the elements stored inside the dual chamber cartridge can only flow through said cannula.

In this connection it should be noted that said seal may be moveably arranged in a distal carriage reception space relative to the cannula. By arranging said seal moveable in said distal carriage reception space, the seal can move relative to the cannula upon activation of the autoinjector when the dual chamber cartridge is moved towards the cannula carriage. It may thus be possible that said seal moves from a distal end of the cannula carriage reception space towards a proximal end of said cannula carriage reception space upon activation of the autoinjector.

According to a further embodiment the cannula carriage comprises one or more detents at a distal end thereof. Said detents may be configured to hold the dual chamber cartridge, i.e. to secure the dual chamber cartridge at the cannula carriage.

It may further be possible that the cannula carriage is in contact with the dual chamber cartridge via the one or more detents in the storage state of the autoinjector. Said detents may for example be configured to secure the cannula carriage at the dual chamber cartridge. Additionally or alternatively, they may be configured to hold the cannula carriage at a predetermined distance with respect to the dual chamber cartridge.

Furthermore, on activation of the autoinjector, the dual chamber cartridge may be configured to overcome said one or more detents such that the cannula pierces the septum. That is, the one or more detents may be configured to provide a securing mechanism that has to be overcome upon activation of the autoinjector.

It could further be possible that a movement of the dual chamber cartridge towards the cannula carriage moves said seal towards the cannula to pierce said seal on activation of the autoinjector.

In this connection it should also be noted that the movement of the dual chamber cartridge towards the cannula carriage may move said seal towards the cannula to first pierce said seal and then secondly pierce said septum to allow the cannula to extend beyond said seal and said septum into the dual chamber cartridge.

According to another embodiment of the invention the autoinjector further comprises a distal stopper arranged in said dual chamber cartridge, and a piston rod, wherein, on activation of said autoinjector, a movement of said piston rod towards the distal stopper brings about a movement of said dual chamber cartridge towards said cannula carriage. That is, to activate the autoinjector a user may move the piston rod towards said distal stopper which then translates into a movement of the dual chamber cartridge towards the cannula carriage.

It may further be possible that once said septum is pierced, a movement of said distal stopper within the dual chamber cartridge is facilitated. Such a movement of the distal stopper may for example urge the elements stored in the dual chamber cartridge towards the cannula in order to be dispensed, i.e. injected into a patient.

According to another embodiment of the invention the dual chamber cartridge comprises a proximal and a distal stopper and a bypass passage. Said proximal and distal stoppers may have various tasks. For example, the distal stopper can be configured to be moved towards the proximal stopper upon activation of the autoinjector.

Furthermore, it may possible that the proximal stopper is configured to urge the elements that are stored in the autoinjector towards the cannula upon a dispensing motion of the autoinjector, in particular together with the distal stopper.

The proximal and the distal stopper may be arranged at a distance from one another in a storage state in order to provide two chambers in which the elements of the medicament can be stored separately from one another.

The bypass passage may be arranged at or near the proximal stopper such that upon activation of the autoinjector said proximal stopper may move towards said bypass passage such that the element stored behind, i.e. distally relative to the proximal stopper can flow through said bypass passage to bypass the proximal stopper and to reach the element that is stored in front of, i.e. proximally relative to the proximal stopper in order to be mixed with one another.

It may further be possible that the dual chamber cartridge comprises a first chamber more proximally arranged than a second chamber, wherein the first chamber is filled with a solid material of said medicament and the second chamber is filled with a liquid material of said medicament.

According to one embodiment the first chamber is separated from the second chamber by the proximal stopper. That is, in this case no additional separation walls or the like are necessary to separate the first chamber from the second chamber.

In this connection it should be noted that in some cases the autoinjector further comprises a piston rod configured to act on said distal stopper and said proximal stopper and to thereby move the proximal stopper such that it is arranged at the bypass passage for urging the liquid material into the first chamber past the proximal stopper via the bypass passage such that the liquid material can be mixed with the solid material.

According to a further aspect of the invention a method of activating an autoinjector is provided, the autoinjector comprising a dual chamber cartridge comprising first and second elements of a medicament, with the first and second elements each being stored in a separate chamber of the dual chamber cartridge and a cannula carriage having a cannula and a piston rod. The method according to the invention comprises the steps of: - moving the piston rod proximally towards a distal stopper arranged in the dual chamber cartridge and thereby moving said dual chamber cartridge towards the cannula carriage to pierce a septum of said dual chamber cartridge with said cannula.

In some cases the method may further comprise the step of allowing air to be expelled from said dual chamber cartridge via said cannula and then further moving the distal stopper towards a proximal stopper to thereby urge a second element of the medicament from a second chamber into a bypass passage to bypass the proximal stopper such that the second element comes into contact with the first element in the first chamber arranged between the cannula carriage and the proximal stopper.

According to a further aspect of the invention an autoinjector comprising a dual chamber cartridge comprising two elements of a medicament that are each stored in a separate chamber of the dual chamber cartridge is provided. The dual chamber cartridge further comprises a bypass passage and an outer cap, an inner cap and a cannula shield covering a cannula of the autoinjector, wherein, on activation of the autoinjector said inner cap is removable from the autoinjector before said outer cap is removed from said autoinjector.

That is, according to this further aspect of the invention the autoinjector comprises a dual chamber cartridge comprising two chambers configured to store two elements of a medicament, i.e. one medicament in each chamber. Furthermore, the autoinjector comprises a bypass passage that may be configured to enable the two elements to be mixed with one another when a mixing mechanism is triggered.

The autoinjector according to the invention further comprises an outer cap, an inner cap and a cannula shield covering a cannula of the autoinjector. On activation of the autoinjector said inner cap is removable from the autoinjector before said outer cap is removed from said autoinjector.

Said inner and outer caps may have the same or different functions. For example they may both be configured to shield a user from the cannula when the autoinjector is in the storage state. Additionally or alternatively either one of the inner and the outer cap or both may be configured to keep the autoinjector in a storage state to prevent premature mixing and/or dispensing of the elements stored in the dual chamber cartridge.

It can for example be possible that the inner cap is configured to prevent premature mixing of the elements stored in the dual chamber cartridge while the outer cap is configured to prevent premature dispensing of the elements.

The cannula shield can further be configured to shield the cannula and/or to ensure a sterile environment for the cannula in the storage stage of the autoinjector.

Additionally, the cannula shield can also be configured to keep the autoinjector from prematurely mixing and dispensing the elements stored therein, for instance. In this connection it should be noted that it would also be possible to complete mixing and to accept a certain degree of over pressure in the proximal chamber of the syringe.

According to an embodiment of the invention the autoinjector is configured such that removal of the inner cap simultaneously brings about a removal of the cannula shield such that only one action is required from a user to remove both the inner cap and the cannula shield to start an activation of the autoinjector. In this connection it should be noted that the mixing process of the two materials should be avoided prior to removal of the inner cap, hence the reconstitution or mixing process is preferably hindered prior to removal of the inner cap, so that removal of the inner cap is considered a first stage of activation of the autoinjector.

In this connection it should be noted that the inner cap may comprise a grip projecting from the autoinjector to facilitate the removal of the inner cap for the user.

The grip may comprise a ring which can be easily gripped by a user.

According to another embodiment removal of the inner cap permits a movement of air out of the dual chamber cartridge. This may facilitate the mixing of the two elements stored in the dual chamber cartridge.

According to another embodiment of the invention the inner cap comprises a cannula shield holder at an end of the inner cap disposed opposite to a base of the inner cap such that the inner cap may be configured to additionally hold a cannula shield.

According to a further embodiment the inner cap comprises inwardly facing projections that engage the cannula shield. This may for example be advantageous in case the cannula shield is supposed to be removed together with the inner cap as said projections can safely secure the inner cap to the cannula shield.

Said inwardly facing projections may for example be integrally formed with the cannula shield holder of the inner cap. This can lead to lower production costs and time as less single components are needed to form the inner cap, the inwardly facing projections and the cannula shield holder. Thus, one could even think of providing an inner cap, cannula shield holder and inwardly facing projections that are made as one-piece unit. Alternatively, only the cannula shield holder and the projections could be made as a single unit, for instance.

In this connection it should further be noted that the inwardly facing projections may also be formed by a metal insert received within the cannula shield holder of the inner cap which may have benefits in view of durability and strength of the inwardly facing projections.

According to a further embodiment the outer cap is configured to receive a part of a cannula shield of the autoinjector.

According to yet another embodiment an axial movement of a cannula guard of the auto-injector in the direction of the dual chamber cartridge brings about a release of a drive mechanism of a plunger of the dual chamber cartridge for dispensing a medicament stored in the dual chamber cartridge.

The devices and mechanisms according to the present disclosure may be configured to store and subsequently dispense a drug or, used synonymously herein, a medicament.

According to the present disclosure, a drug or a medicament is a pharmaceutical formulation that contains one or more active pharmaceutical ingredients. An active pharmaceutical ingredient is a substance that is biologically active or has a biological effect on humans or animals. It is used in the diagnosis, mitigation, cure, treatment or prevention of a medical condition or to otherwise affect the structure or function of the body of humans or animals.

Active pharmaceutical ingredients, medicaments and drugs that may be contained and/or dispensed by the mechanisms and devices according to the present disclosure are, inter alia, described in handbooks such as Merck Index, for example 15th edition, Rote Liste, such as Rote Liste 2023, the publication Approved Drug Products with Therapeutic Equivalence Evaluations (Orange Book) by the U.S. FDA and the Purple Book Database of Licensed Biological Products, also by the U.S. FDA, all of which are freely accessible via the Internet.

Active pharmaceutical ingredients, medicaments and drugs that may be contained and/or dispensed by the mechanisms and devices according to the present disclosure may be selected from: analgesics and antirheumatics (main group 05 of Rote Liste); anti-obesity drugs (main group 06 of Rote Liste); antiallergics (main groups 07 of Rote Liste); antiane-mics (main group 08 of Rote Liste); antibiotics (main group 10 of Rote Liste); antidiabetic drugs (main group 12 of Rote Liste); antidote medications (main group 13 of Rote Liste); antihemorrhagics (antifibrinolytics and other hemostatic agents) (main group 16 of Rote Liste); anti hypoglycemic drugs (main group 18 of Rote Liste); antihypotonics and agents for shock therapy (main group 19 of Rote Liste); anticoagulants (main group 20 of Rote Liste); beta-receptor blockers, calcium channel blockers and inhibitors of the renin-angiotensin-aldosterone system (main group 27 of Rote Liste); bronchospasmolytics/antiasthmatics and other agents for the respiratory tract (main group 28 of Rote Liste); dermatics (main group 32 of Rote Liste); diagnostic agents and agents for diagnostic preparation (main group 35 of Rote Liste); enzyme inhibitors, preparations for enzyme deficiency and transport proteins (main group 40 of Rote Liste); gene and cell therapeutics and RNA interference therapeutics (main group 42 of Rote Liste); hypophysial and hypothalamic hormones, other regulatory peptides, their inhibitors and analogues (main group 50 of Rote Liste); immunomodulators (main group 51 of Rote Liste); lipid-lowering drugs (main group 58 of Rote Liste); migraine medication (main group 61 of Rote Liste); neuropathy preparations and other neurotropic agents (main group 66 of Rote Liste); ophthalmics (main group 67 of Rote Liste); osteoporosis agents, calcium/bone metabolism regulators (main group 68 of Rote Liste); psychotropic drugs (main group 71 of Rote Liste); sera, immunoglobulins and vaccines (main group 75 of Rote Liste); sex hormones and their inhibitors (main group 76 of Rote Liste); vitamins (main group 84 of Rote Liste); oncology drugs, cytostatics, other antineoplastic agents and protectives (main group 86 of Rote Liste); anaesthetics; autoimmune disorder medication, such as rheumatoid arthritis medication and/or multiple sclerosis medication; and/or cardiovascular disease medication.

In a specific example, an active ingredient of a medicament delivered by the mechanisms and devices according to the present disclosure is an antirheumatic.

In a further specific example, an active ingredient of a medicament delivered by the mechanisms and devices according to the present disclosure is an anti-obesity drug.

In a further specific example, an active ingredient of a medicament delivered by the mechanisms and devices according to the present disclosure is an antiallergic.

In a further specific example, an active ingredient of a medicament delivered by the mechanisms and devices according to the present disclosure is an antibiotic.

In a further specific example, an active ingredient of a medicament delivered by the mechanisms and devices according to the present disclosure is an antidiabetic drug.

In a further specific example, an active ingredient of a medicament delivered by the mechanisms and devices according to the present disclosure is an antihypoglycemic drug.

In a further specific example, an active ingredient of a medicament delivered by the mechanisms and devices according to the present disclosure is an enzyme inhibitor. In a further specific example, an active ingredient of a medicament delivered by the mechanisms and devices according to the present disclosure is a preparation for enzyme deficiency. In a further specific example, an active ingredient of a medicament delivered by the mechanisms and devices according to the present disclosure is a transport protein.

In a further specific example, an active ingredient of a medicament delivered by the mechanisms and devices according to the present disclosure is a gene therapeutic. In a further specific example, an active ingredient of a medicament delivered by the mechanisms and devices according to the present disclosure is a cell therapeutic. In a further specific example, an active ingredient of a medicament delivered by the mechanisms and devices according to the present disclosure is an RNA interference therapeutics.

In a further specific example, an active ingredient of a medicament delivered by the mechanisms and devices according to the present disclosure is a hypophysial hormone. In a further specific example, an active ingredient of a medicament delivered by the mechanisms and devices according to the present disclosure is a hypothalamic hormone. In a further specific example, an active ingredient of a medicament delivered by the mechanisms and devices according to the present disclosure is a regulatory peptide other than a hypophysial hormone or a hypothalamic hormone.

In a further specific example, an active ingredient of a medicament delivered by the mechanisms and devices according to the present disclosure is an inhibitor of a hypophysial hormone. In a further specific example, an active ingredient of a medicament delivered by the mechanisms and devices according to the present disclosure is an inhibitor of a hypothalamic hormone. In a further specific example, an active ingredient of a medicament delivered by the mechanisms and devices according to the present disclosure is an inhibitor of a regulatory peptide other than a hypophysial hormone or a hypothalamic hormone.

In a further specific example, an active ingredient of a medicament delivered by the mechanisms and devices according to the present disclosure is an analogue of a hypophysial hormone. In a further specific example, an active ingredient of a medicament delivered by the mechanisms and devices according to the present disclosure is an analogue of a hypothalamic hormone. In a further specific example, an active ingredient of a medicament delivered by the mechanisms and devices according to the present disclosure is an analogue of a regulatory peptide other than a hypophysial hormone or a hypothalamic hormone.

In a further specific example, an active ingredient of a medicament delivered by the mechanisms and devices according to the present disclosure is an immunomodulator.

In a further specific example, an active ingredient of a medicament delivered by the mechanisms and devices according to the present disclosure is a lipid-lowering drug.

In a further specific example, an active ingredient of a medicament delivered by the mechanisms and devices according to the present disclosure is an osteoporosis agent.

In a further specific example, an active ingredient of a medicament delivered by the mechanisms and devices according to the present disclosure is a calcium metabolism regulator.

In a further specific example, an active ingredient of a medicament delivered by the mechanisms and devices according to the present disclosure is a bone metabolism regulator.

In a further specific example, an active ingredient of a medicament delivered by the mechanisms and devices according to the present disclosure is a sex hormone.

In a further specific example, an active ingredient of a medicament delivered by the mechanisms and devices according to the present disclosure is an inhibitor of a sex hormone.

In a further specific example, an active ingredient of a medicament delivered by the mechanisms and devices according to the present disclosure is an autoimmune disorder medication.

In a further specific example, an active ingredient of a medicament delivered by the mechanisms and devices according to the present disclosure is a rheumatoid arthritis medication.

In a further specific example, an active ingredient of a medicament delivered by the mechanisms and devices according to the present disclosure is a multiple sclerosis medication.

In a further specific example, an active ingredient of a medicament delivered by the mechanisms and devices according to the present disclosure is a cardiovascular disease medication.

The drug or medicament may have, for example, one or more active pharmaceutical ingredients selected from one or more of: carbohydrates and polysaccharides; polypeptides, peptides and proteins (e.g., antibodies, antibody fragments, hormones, growth factors, and enzymes); small molecules with a molecular weight of 500 Da or less; and nucleic acids, double or single stranded DNA (including cDNA and naked DNA), RNA, antisense nucleic acids, such as antisense DNA and RNA, small interfering RNA (siRNA), ribozymes, genes, and oligonucleotides. Nucleic acids may be included into molecular delivery systems such as liposomes, vectors, or plasmids.

The drugs or medicaments contained in or dispensed with the mechanisms according to the present disclosure may comprise active pharmaceutical ingredients used in the treatment and/or mitigation and/or prevention and/or cure and/or diagnosis of many different types of medical conditions. These may include, inter alia, one or more of the following conditions: acute coronary syndrome, angina, myocardial infarction, anaphylactic shock, atherosclerosis, diabetes mellitus or complications associated with type 1 or type 2 diabetes mellitus such as diabetic retinopathy, cancer, macular degeneration, inflammation, hay fever, rheumatoid arthritis, thromboembolism disorders such as deep vein or pulmonary thromboembolism, infertility, growth disorder, migraine, autoimmune disorders, multiple sclerosis, osteoporosis, allergies, obesity, and/or cardiovascular diseases.

With a specific example, the condition may be diabetes mellitus.

With another specific example, the condition may be a complication associated with type 1 diabetes mellitus. With another specific example, the condition may be a complication associated with type 2 diabetes mellitus. With another specific example, the condition may be diabetic retinopathy.

With another specific example, the condition may be an inflammation.

With another specific example, the condition may be rheumatoid arthritis.

With another specific example, the condition may be infertility.

With another specific example, the condition may be growth disorder.

With another specific example, the condition may be migraine.

With another specific example, the condition may be an autoimmune disorder.

With another specific example, the condition may be multiple sclerosis.

With another specific example, the condition may be osteoporosis.

With another specific example, the condition may be an allergy.

With another specific example, the condition may be obesity.

With another specific example, the condition may be a cardiovascular disease.

For example, the active pharmaceutical ingredients for the therapy of type 1 or type 2 diabetes mellitus or complications associated with type 1 or type 2 diabetes mellitus include an insulin, such as, human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof.

The active pharmaceutical ingredient may also be a hormone, such as a hypophysis hormone or a hypothalamus hormone or a regulatory active peptide and an antagonist of a regulatory active peptide. The hormone may be a fertility hormone, a gonadotropine, a growth hormone, a steroid hormone, such as testosterone or oestrogen, a parathyroid hormone, such as teriparatide, epinephrine or a follicle-stimulating hormone.

The active pharmaceutical ingredient may also be an oligonucleotide.

The active pharmaceutical ingredient may also be an antibody. The term "antibody" includes an immunoglobulin molecule or an antigen-binding portion, such as a fragment antigen-binding region, of an immunoglobulin molecule, an antigen-binding molecule based on tetravalent bispecific tandem immunoglobulins, and/or a dual variable region antibody-like binding protein having cross-over binding region orientation (CODV). The antibody may be a monoclonal, a polyclonal, a recombinant, or a chimeric antibody. It may be a deimmunized or humanized, a fully human, or a non-human, such as a murine, antibody. The antibody may be a single chain antibody.

The active pharmaceutical ingredient may also be a polysaccharide, such as a glucosa-minoglycane, a hyaluronic acid, a heparin, a low molecular weight heparin or an ultra-low molecular weight heparin or a derivative thereof. The active pharmaceutical ingredient may also be a sulphated polysaccharide, e.g. a poly-sulphated form of the above-mentioned polysaccharides, and/or a pharmaceutically acceptable salt thereof.

The drug or medicament contained in and/or dispensed with the mechanisms and devices according to the present disclosure may include one or more of the following active pharmaceutical ingredients: abatacept, adalimumab, aflibercept, alirocumab, anakinra, apomorphine, aripiprazole, belimumab, benralizumab, betamethasone, bimekizumab, brodalumab, brolucizumab, buprenorphine, certolizumab pegol, choriogonadotropin alfa, cyanocobalamin, carbepoetin alfa, denosumab, dulaglutide, dupilumab, enoxaparin sodium, epinephrine, epoetin, epoetin alfa, epoetin beta, epoetin theta, epoetin zeta, erenumab, etanercept, evolocumab, exenatide, filgrastim, follitropin, follitropin alfa, follitropin delta, folic acid, fondaparinux, fremanezumab, fulvestrant, gadobenic acid, gadobutrol, gadodiamide, gadoteric acid, gadoxetic acid, galcanezumab, ganirelix, glatiramer, glucagon, golimumab, goserelin, guselkumab, heparin, icatibant, inclisiran, infliximab, inotersen, insulin aspart, insulin degludec, insulin detemir, insulin glargine, insulin glulisine, insulin human, insulin human-isophane, insulin lispro, insulin isophane, interferon beta-1a, ixekizumab, lanadelumab, lanreotide, lebrikizumab, leuprorelin, liraglutide, lonapegsomatropin, lutropin alfa, menotropin, mepolizumab, methotrexate, methoxy-polyethylene glycol-epoetin beta, mirikizumab, moroctocog alfa, natalizumab, nirsevimab, ofatumumab, omalizumab, paliperidone, pegfilgrastim, peginterferon alfa-2a, peginterferon beta-1a, pegvaliase, propofol, pyridoxine, ranibizumab, rho(D) immune globulin, risankizumab, romosozumab, ropeginterferon alfa-2b, sarilumab, satralizumab, secukinumab, semaglutide, somapacitan, somatrogon, somatropin, sumatriptan, teriparatide, tezepelumab, tildrakizumab, tinzaparin sodium, tocilizumab, tralokinumab, triptorelin, ustekinumab, vedolizumab, volanesorsen, and vutrisiran.

Additionally or alternatively, the drug or medicament contained in and/or dispensed with the mechanisms and devices according to the present disclosure may include one or more of the following active ingredients: Lixisenatide, Taspoglutide, Albiglutide, Efpeglenatide, GLP-1 Eligen, Nodexen, Pegapamodtide, Tirzepatide, and Bamadutide.

Additionally or alternatively, the drug or medicament dispensed with the mechanisms and devices according to the present disclosure may include one or more of the following active ingredients: Mipomersen sodium, Linagliptin, Vildagliptin, Sitagliptin, Denagliptin, Sax-agliptin, Berberine, Lutropin, Choriongonadotropin, Desmopressin, Terlipressin, Gonadorelin, Buserelin, Nafarelin, Hylan G-F 20.

Additionally or alternatively, the drug or medicament dispensed with the mechanisms and devices according to the present disclosure may include as an active ingredient a piperidine alkaloid, such as azimine, azithromycine, azcarpine, and/or carpaine.

The drug or medicament may also contain one or more of the following: pharmaceutically acceptable solvents or salts of the active pharmaceutical ingredients described herein; analogues and derivates of the active pharmaceutical ingredients described herein; biosimilars of the active pharmaceutical ingredients described herein; and pharmaceutically acceptable carriers of the active pharmaceutical ingredients described herein.

The drug or medicament contained in and/or dispensed with the mechanisms and devices according to the present disclosure may include one or more of the following active pharmaceutical ingredients: paracetamol (acetaminophen), nonsteroidal anti-inflammatory drugs, such as aspirin, ibuprofen and naproxen, cox-2 inhibitors, such as rofecoxib, celecoxib, and etoricoxib, opioids, such as morphine, codeine, oxycodone, hydrocodone, dihydromorphine, pethidine, alcohols, cannabis, nefopam, flupirtine, ziconotide.

The drug or medicament may be contained in a container from which it is dispensed by an action of a user. The container may, for example, be a cartridge, a reservoir or a syringe. The container may extend between a dispensing end and a rear end of the container. The container may be configured to dispense the drug or medicament at the dispensing end, for example through a pierceable septum. At the rear end, the container may be sealed to prevent the exit of the drug or medicament. For example, the container may be sealed by a flexible and/or displaceable plunger at the rear end. In between the dispensing end and the rear end, the container may have a generally tubular hollow housing.

The container may have a single compartment for storing the drug or medicament. Alternatively, the container may also have more than one compartment, such as two compartments. Different compartments thereby may comprise different substances, such as drugs, medicaments, active ingredients, solvents or carriers. Different compartments may also comprise a part of the medicament or drug in solid form, for example as a powder, such as a lyophilized powder, and another part of the medicament in liquid form. The solid part may, for example, comprise the one or more active pharmaceutical ingredient and/or the liquid part may be a solvent.

The container may be configured to allow a mixing of the contents from at least two of the compartments, such as from all of the compartments, prior to and/or during dispensing of the medicament or drug. For example, the container may be configured to provide a fluid connection between the at least two, such as between all of the compartments, for mixing.

For example, the container may comprise two compartments, one comprising a first part of the drug or medicament in solid form, for example the one or more active ingredients, and the other one comprising a second part of the drug or medicament in liquid form, such as a solvent. The container may be configured to provide a fluid connection between the two compartments and to mix the first and second parts prior or during dispensing. This may reconstitute the drug or medicament from its solid form.

The devices and mechanisms according to the present disclosure may be disposable, which means that the container holding the medicament is non-replaceable or non-refillable and the devices are disposed of after having dispensed a last dose from the container. Alternatively, they may be reusable, for example, they may allow to replace a container by a new container or to refill the container.

The devices and mechanisms according to the present disclosure may be fixed dose devices and mechanisms that only allow the dispensing of a fixed dose of the medicament or drug. Alternatively, they may be variable dose devices and mechanisms that allow for setting of a dose from a multitude of differing settable doses.

The mechanisms and devices according to the present disclosure may comprise a dose definition mechanism that allows a user to set the dose to be dispensed prior to dispensing. The dose thereby may be settable as a fixed dose only, for example for fixed dose devices or mechanisms. The dose also may be settable from a multitude of settable doses, such as for variable dose devices or mechanisms.

The doses may be settable as integer multiples of international units of the drug or medicament. They also may be settable as integer multiples of a fraction of said international units, such as integer multiples of half units.

The mechanisms and devices according to the present disclosure may be single dose devices that allow dispensing of a dose from the container only once, so that the container is a single dose container. They also may be multiple dose devices that allow for a sequential dispensing of more than one dose from the same container, so that the container is a multi-dose container.

The drug delivery devices according to the present disclosure may be injection devices, such as needle-based injection devices.

Features and functions that may be implemented in mechanisms and devices according to the present disclosure, and/or requirements that may be met by those mechanisms and devices are also described in standard ISO 11608-1 (Needle-based injection systems for medical use - Requirements and test methods - Part 1: Needle-based injection systems), such as ISO 11608-1:2022(E), and standard ISO 11608-5 (Needle-based injection systems for medical use - Requirements and test methods - Part 5: Automated functions), such as ISO 11608-5:2022(E).

The invention is further described by the following embodiments and Figures which show:
- Fig. 1:: various sectional views of different stages of use of a dual chamber autoinjector;
- Fig. 2: a sectional view of a pre-filled syringe including a cannula shield;
- Fig. 3:: a sectional view of the pre-filled syringe of Fig. 2, without the cannula shield;
- Fig. 4:: a sectional view of a dual chamber cartridge comprising a cannula carriage and a cannula shield;
- Fig. 5:: a sectional view of the dual chamber cartridge of Fig. 4 without the cannula shield;
- Fig. 6:: a close-up view of the cannula carriage of Figs. 4 and 5;
- Fig. 7:: detailed views of the outer and inner caps of the autoinjector;
- Fig. 8:: detailed views of a drive chassis;
- Fig. 9:: detailed views of a first kind of piston rod;
- Fig. 10:: detailed views of an interaction between the drive chassis of Fig. 8 with the piston rod of Fig. 9;
- Fig. 11:: detailed views of a sleeve;
- Fig. 12:: detailed views of the inner cap of Fig. 7;
- Fig. 13:: detailed views of parts of a first kind of autoinjector;
- Fig. 14:: detailed views of a dial;
- Fig. 15:: detailed views of a second kind of piston rod;
- Fig. 16:: detailed views of parts of a second kind of autoinjector;
- Fig. 17:: detailed views of a first kind of cannula;
- Fig. 18:: detailed views of a second kind of cannula

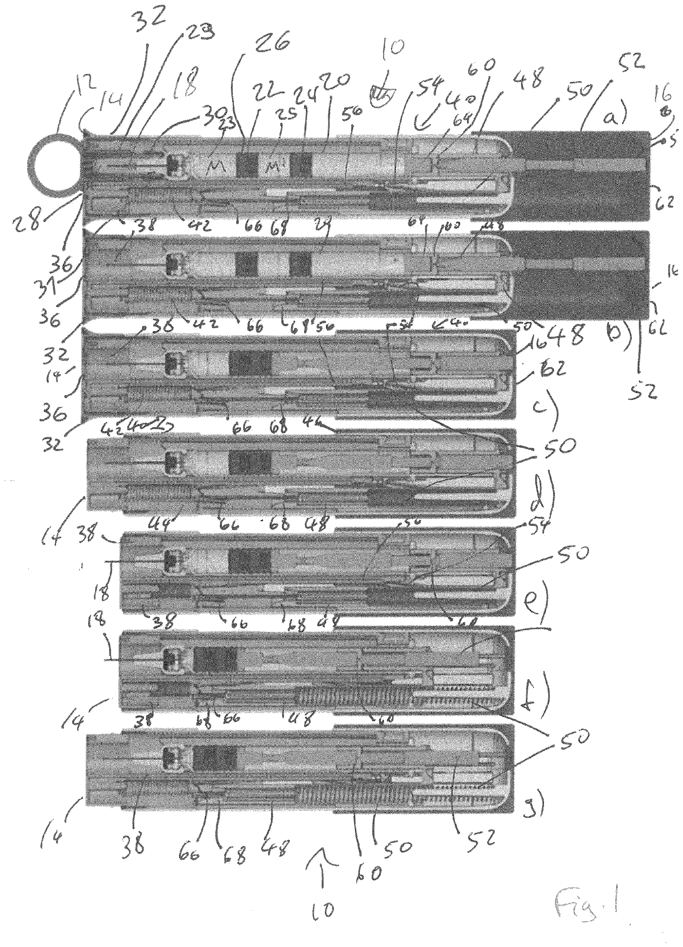

Figs. 1a to g show various sectional views of different stages of use of a dual chamber autoinjector 10. Fig. 1a shows the as delivered state of the autoinjector 10, with a handle 12 protruding from a proximal end 14 of the autoinjector. The proximal end 14 is oppositely disposed to the distal end 16.

In this connection it should be noted that the terms proximal and distal refer to the position of a cannula 18 of the autoinjector 10 relative to a patient (not shown) with proximal meaning closest to a main mass of the body of a patient and distal meaning it is more distant from the main mass of the body of a patient.

Hence, e.g. the proximal end 14 of the autoinjector 10 is that end that is placed at a patients skin with the distal end 16 being remote from the patient's skin in use, i.e. the end of the device having a needle that pierces a patient's skin is the proximal end 14.

The cannula 18 is arranged at a pre-filled syringe 20. The pre-filled syringe is a so-called dual chamber pre-filled syringe 20 which in a storage state comprises two chambers 23, 25 separated from one another for storage of two kinds of material M, M' by a proximal stopper 22, with the second chamber 25 also being bound by a distal stopper 24.

Such a pre-filled syringe and hence the autoinjector 10 can be used for mixing, e.g. liquid/liquid compositions, respectively reconstitution processes, e.g. powder/liquid compositions, in which the two different materials M, M' are stored separate from one another but are mixed in a mixing or a reconstitution process to form a medicament before dispensing of the medicament. The reason for this is that the medicament may not have a long storage life once mixed but its components do.

The two chambers can be connected one to another via a bypass passage 26 once the distal stopper and the proximal stopper are moved proximally towards the cannula 18.

The cannula 18 is covered by a cannula shield 28 that can be removed from the pre-filled syringe 20 on removal of the handle 12 from a cap 32 present at the proximal end 14 of the autoinjector 10.

The handle 12 comprises an inner cap 34 attached thereto, with the inner cap 34 being arranged within the cap 32. The inner cap 34 is configured to hold and move a metal pressing 30 arranged to hold and remove the cannula shield 28 on removal of the handle 12 from the cap 32.

For this purpose the cap 32 comprises an aperture 36 via which the inner cap 34, the metal pressing 30 and the cannula shield 28 can be removed from the autoinjector 10 and the pre-filled syringe 20 via the aperture 36.

The autoinjector 10 further comprises a cannula guard 38 arranged at the proximal end 14. The cannula guard 38 is configured to be moved relative to the cannula 18 once the cap 32 is removed during an activation operation and a lock out operation as indicated in Figs. 1c to g.

The cannula guard 38 is arranged to move relative to a housing 40 of the autoinjector 10. A lock-out spring 42 is arranged between the cannula guard 38 and the housing 40. In the state shown in Figs. 1a to d and g, the lock out 42 spring is in its extended state whereas in the state indicated in Fig. 1e and f, the lock-out spring 42 is in the compressed state. The housing 40 is formed by an inner body 44 and an outer body 46, with the pre-filled syringe being held at the inner body 44 which in turn is snap fit into place at the outer body 46 in such a manner that the pre-filled syringe cannot move axially with respect to either of the inner body 44 and the outer body 46.

The autoinjector 10 further comprises a drive chassis 48 arranged movable in the housing 40 between a storage position (Fig. 1a) respectively a pre-dispense position (Figs. 1b to e) and a dispensed position (see Figs. 1f and g).

A drive spring 50 is biased between the outer body 46 and the drive chassis 48 and is configured to release energy in order to drive the drive chassis from the pre-dispense position to the dispensed position once the drive chassis 48 has been released for movement.

The drive chassis 48 is released for movement relative to the housing 40 once the cannula guard 38 is moved distally (see e.g. Figs. 1e and f) and a cannula guard plunger 56 engages trigger arms 54 of the drive chassis 48 and releases an engagement between the trigger arms 54 and the housing 40.

The spring chassis 48 then entrains a piston rod 52 in order to dispense a medicament composed of the mixed two kinds of material M, M'.

The autoinjector 10 further comprises a sleeve 62 within which the housing 40 is received and which is moveable relative to the housing 40. The sleeve 62 comprises a piston rod holder 58 for releasably holding the piston rod 52.

A front end 64 of the piston rod 52 is initially held at a journal 60 present at the drive chassis 48. The piston rod 52 is entrained by the journal 60 on carrying out the dispensing action. This entraining action is carried out by the drive chassis 48 engaging a middle portion of the piston rod 52 during the dispensing action.

The inner body 44 comprises an end of dose click arm 66 that is configured to engage the drive chassis 48 towards an end of dose following the carrying out of the dispensing action. The end of dose click arm 66 cooperates with an end of dose click ramp 68 provided at the drive chassis 48. The end of dose click arm 66 can only engage the drive chassis once the drive chassis 48 has moved proximally.

Fig. 1b shows a state of the autoinjector 10 in which the inner cap 34 and the cannula shield 28 are removed prior to a reconstitution process having been carried out.

Fig. 1c shows a state in which the sleeve 62 is moved relative to the housing 40 in order to move the distal plunger 24 and the second medicament M' proximally such that the second medicament M' can come into contact with the first medicament via the bypass passage 26 to carry out the reconstitution process.

Fi. 1d shows the state in which the cap 32 of the autoinjector 10 has been removed. Only after removal of the cap 32 is it possible to actuate the cannula guard which can be used to activate the autoinjector 10 for the dispensing process.

This occurs as indicated in Fig. 1e when the cannula guard 38 is fully depressed and the cannula guard plunger 56 engages the trigger arms 54 to release the drive chassis 48.

As indicated in Fig. 1f, the cannula guard 38 is still fully depressed and the dispense is completed, i.e. the drive chassis 48 has moved as far proximally as possible.

During this proximal movement of the drive chassis 48, the end of dose click arm 66 engages the end of dose click ramp 68 and emits an audible clicking noise indicating a near completion respectively a completion of the dispensing process.

Once a patient hears the end of dose click, he knows that dispensing is completed and can remove the autoinjector 10 from the point of administration at the patient or start a count to do so, the lock-out spring 42 then moves the cannula guard 38 into a locked position in which the cannula 18 is covered by the cannula guard 38. It is preferred if the cannula guard 38 cannot be moved relative to the cannula 18 when this is in the locked position.

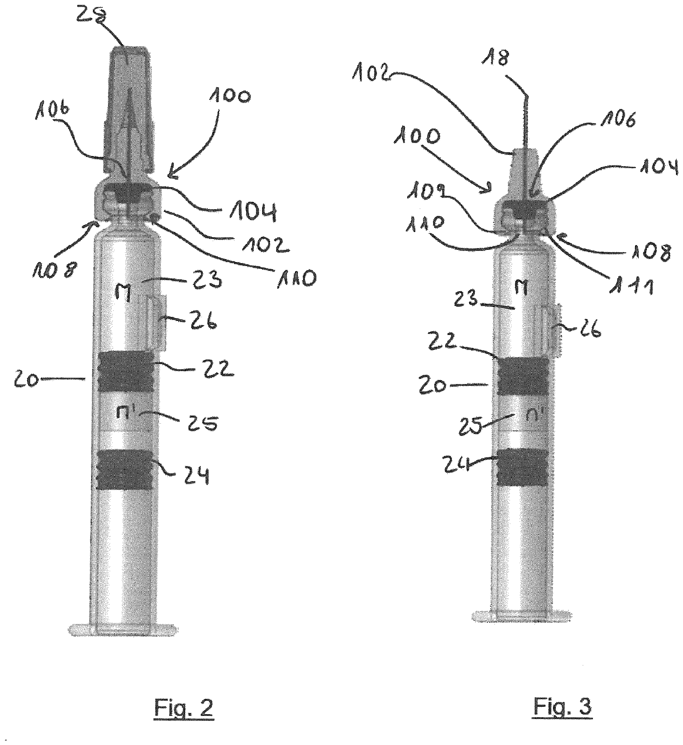

Figs. 2 and 3 show sectional views of a prefilled syringe 20 according to the invention. The syringe 20 of Fig. 2 additionally comprises the removable cannula shield 28 while the syringe 20 shown in Fig. 3 is without said cannula shield 28.

It can be clearly seen that the prefilled syringe 20 comprises a dual chamber cartridge 11 having two chambers 23, 25 wherein the first chamber 23 is arranged proximally relative to the second chamber 25. Each of said two chambers 23, 25 comprises one element M respectively M' of a medicament such that said two elements M, M' are stored separately from one another to enhance the storage time.

It can furthermore be clearly seen that the first and second chambers 23, 25 are separated from one another by the proximal stopper 22 and that the second chamber 25 additionally comprises a distal stopper 24 at its distal end.

The prefilled syringe 20 of Figs. 2 and 3 is shown in the storage state as the proximal stopper 22 is arranged behind, i.e. distally relative to the bypass passage 26 such that the material M' stored in the second chamber 25 cannot yet flow into the first chamber 23.

Additionally, the prefilled syringe 20 of Figs. 2 and 3 comprises a cap and seal assembly 100 having a cap 102 and one or more seals 104 with said cap and seal assembly 100 being fixed to and covering an opening 106 of the dual chamber cartridge 11.

It can be seen that the cap 102 completely surrounds seal 104 as well as the opening 106.

The cannula 18 extends through and beyond the cap and seal assembly 100 as it is depicted in Fig. 2. This way, the elements M, M' stored inside the dual chamber cartridge 11 can only flow through the cannula 18 during the act of dispensing as seal 104 completely seals and covers the potential open residue of the opening 106.

Said cannula 18 can furthermore be stabilized by cap 102 and/or by the one or more seals 104.

The cap and seal assembly 100 of Figs. 2 and 3 further comprises a first flange arrangement 108 while the dual chamber cartridge 11 comprises a second flange arrangement 110 that is shaped complementary to said first flange arrangement. The two flange arrangements 108,110 are engageable with one another such that the cap and seal assembly 100 can safely be secured to the dual chamber cartridge 11.

In the embodiment of Figs. 2 and 3 this is done by providing a first flange arrangement 108 with an undercut 109 and a second flange arrangement 110 with a web 111 such that said undercut 109 can engage with said web 111. In this case it is also possible that the first flange arrangement, i.e. the undercut 109, completely surrounds the second flange arrangement 110, i.e. the web 111.

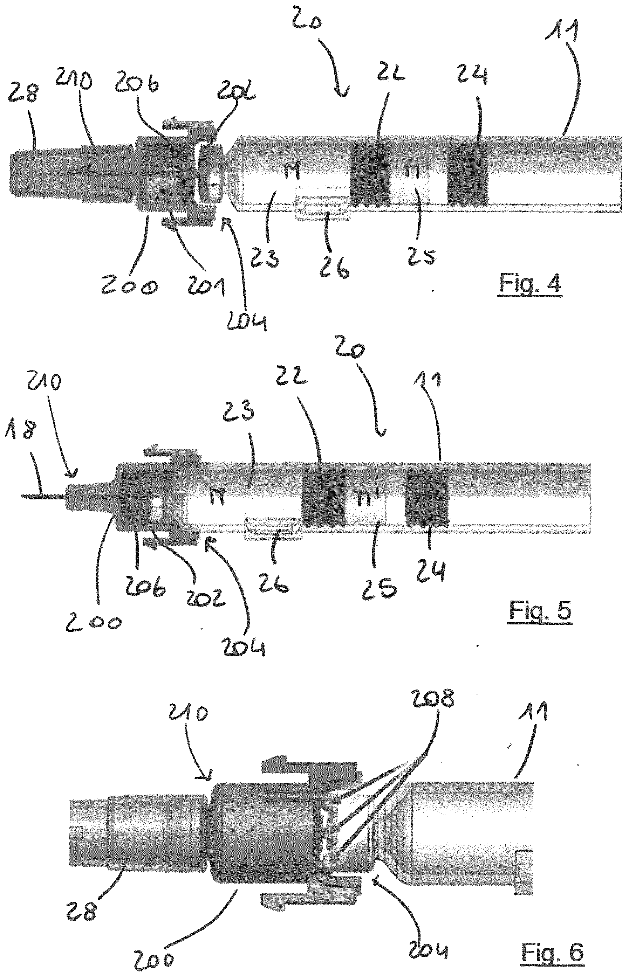

The second aspect of the invention is shown in Figs. 4 to 6 with Figs. 4 and 5 showing detailed sectional views of a prefilled syringe 20 according to this second aspect of the invention and Fig. 6 showing a detailed view of a cannula carriage 200 arranged at said pre-filled syringe 20, in particular at the dual chamber cartridge 11.

In this connection it is noted that for the general features of the prefilled syringe 20 of Figs. 4 to 6 it is referred to the explanations made in connection with Figs. 1 to 4 as said general features are the same. In the following only the features that differ from the embodiments of Figs. 1 to 4 are described.

The prefilled syringe 20 according to Figs. 4 to 6 comprises a cannula carriage 200 instead of the cap and seal assembly 100 of Figs. 2 and 3. Said cannula carriage 200 is arranged at the outlet 106 of the dual chamber cartridge 11.

The cannula 18 is arranged inside of the cannula carriage such that it extends beyond said carriage 200 at a proximal end in the storage state of the autoinjector 10. At a distal end of the cannula carriage 200 said carriage 200 comprises a cannula carriage reception space 201 through which the cannula 18 extends. The cannula 18 is further arranged moveably with respect to said reception space 201 such that upon activation of the autoinjector 10 the dual chamber cartridge 11 is moved towards the cannula carriage 200.

Upon said activation movement the cannula 18 pierces a rubber septum 202 that is arranged at and seals the outlet 106 of the dual chamber cartridge 11 in the storage state. Hence, in the activated state of the autoinjector 10 the cannula carriage 200 is arranged closer to the dual chamber cartridge 11 such that the cannula 18 extends beyond the rubber septum 202 and thus the opening 106 of the dual chamber cartridge 11 into the first chamber 23.

At the distal end 204 of the carriage reception space 201 a seal 206 is arranged moveably with respect to said reception space 201 such that upon the activation process of the autoinjector 10 the seal 206 moves in a proximal direction towards a proximal end of the carriage reception space 201.

The seal 206 is arranged at the carriage reception space 201 to seal the area around the cannula 18. Furthermore, it may be configured to be pierced by the cannula 18 upon activation of the autoinjector 10 such that said seal 206 is pierced before the rubber septum 202 is pierced.

The cannula carriage 200 of Figs. 4 to 6 additionally comprises detents 208 at the distal end 204 thereof through which the cannula carriage 200 is in contact with the dual chamber cartridge 11 in the storage state of the autoinjector 11 (see in particular Fig. 6).

In the activated state of the autoinjector 10 the detents 208 can be overcome such that the cannula carriage 200 moves closer to the dual chamber cartridge 11 (see Fig. 5) to allow the piercing of the septum 202.

Also the prefilled syringe 20 according to Figs. 4 to 6 can comprise an additional cannula shield 28 that is arranged at the proximal end 210 of the cannula carriage 200 in the storage state of the autoinjector 10 (see Fig. 4). Upon activation of the autoinjector 10 the cannula shield 28 can be removed as it is already explained in connection with Fig. 1.

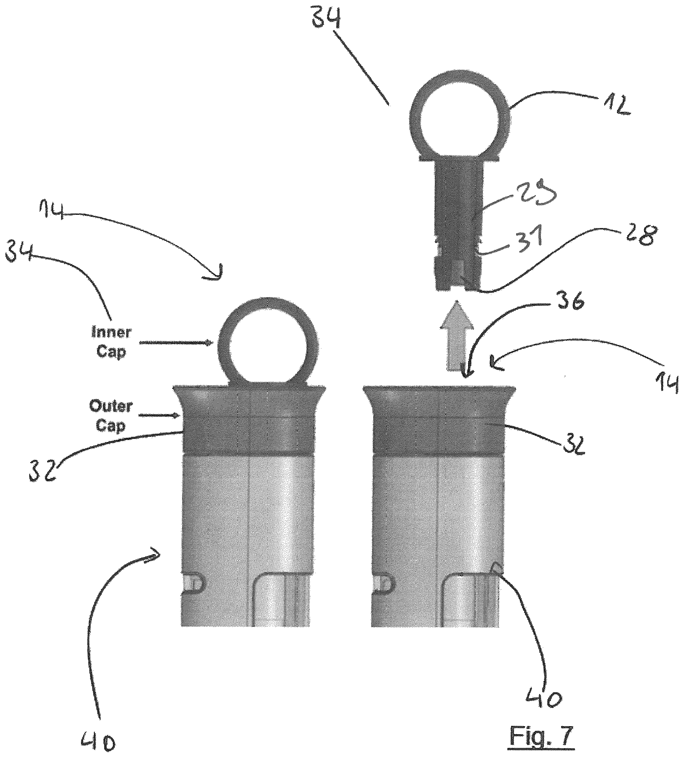

Another aspect of the invention is shown in Fig. 7 which shows a detailed view of the proximal end 14 of the autoinjector 10 comprising an inner and an outer cap 34, 32. The functioning of said inner and outer caps 34, 32 is already described in connection with Fig. 1.

In this connection it is noted that it may additionally be possible that the autoinjector according to Fig. 7, and thus also Fig. 1, additionally comprises the cannula carriage 200 as described in connection with Figs. 4 to 6 and/or the cap and seal assembly 100 as described in connection Figs. 2 and 3.

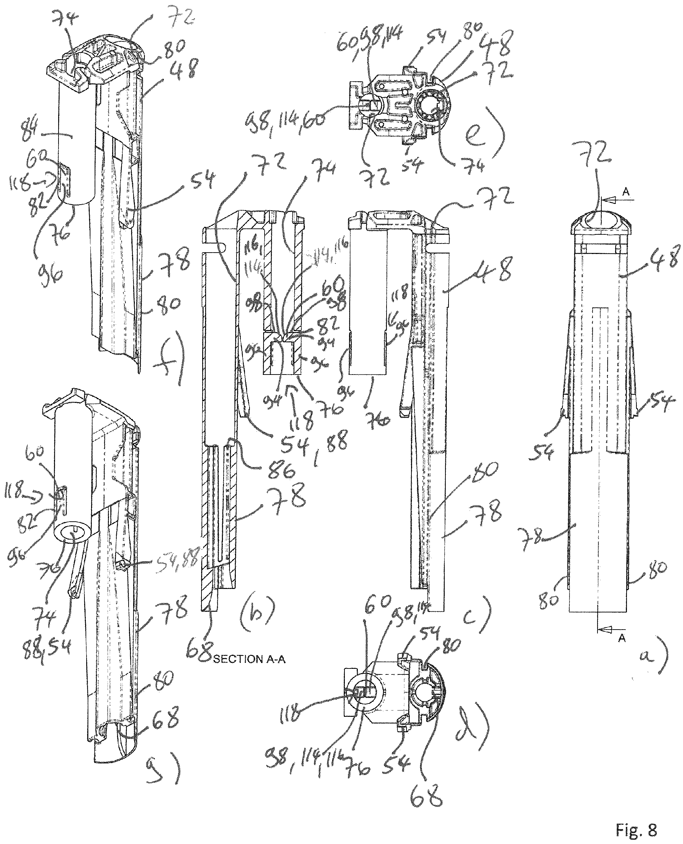

Fig. 8a to 8g show various views of the drive chassis 48. The drive chassis 48 is an at least essentially U-shaped structure having first and second limbs 78, 84, with the second limb 84 being formed shorter than the first limb 78. The first and second limbs 78,84 extend in parallel to one another.

The drive chassis 48 is configured to be linearly driven in the autoinjector 10 by means of the drive spring 50.

For this purpose the second limb 78 is configured as a drive spring receiver 78 having a drive spring reception space 72. In the storage state of the autoinjector 10, the drive spring 50 is compressed in the drive spring reception space 72 between a base 86 of the second limb 78 and the outer body 46 of the autoinjector 10.

The drive chassis 48 is axially held in the storage state via the two trigger arms 54 that project from the second limb 78 and engage a part of the outer body 46.

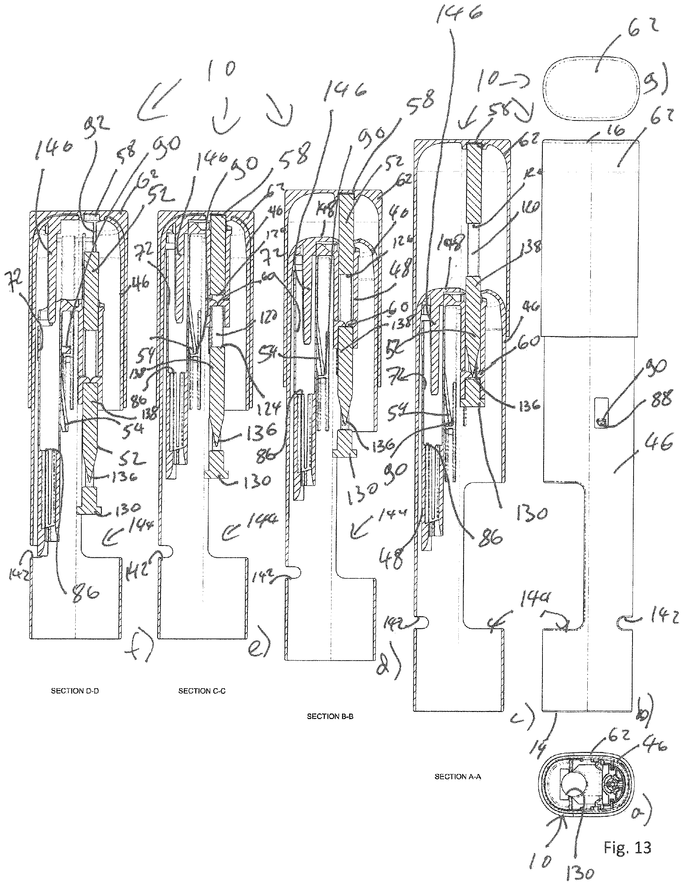

The trigger arms 54 have respective projections 88 that engage a corresponding complementary shaped structure 90 (see Fig. 13) in the storage state of the autoinjector. The drive chassis 48 is also held relative to the outer body 46 during a mixing process.

On carrying out the dispensing operation, the engagement between the respective projections 88 and structures 90 is released and the drive chassis 48 is axially driven proximally by the drive spring 50.

The engagement between the respective projections 88 and structures 90 is released by way of the cannula guard plunger 56 (also known as a needle guard 56).

The respective structures 90 may be formed at windows present in the outer body 46.

The second limb 78 comprises guides 80 via which the linear movement of the drive chassis 48 can be guided relative to the inner and outer body 44,46 of the autoinjector 10.

As indicated in Fig. 8 the guide 80 can be formed by a groove. A corresponding spline 92 interacting with the groove 80 is present in both the inner body 44 and the outer body 46 (see e.g. Fig. 13f with regard to the spline 92 of outer body 46). In this connection it should be noted that the guide 80 of the drive chassis 48 could also be formed by a spline interacting with a correspondingly shaped groove then present at the inner and outer bodies 44, 46.

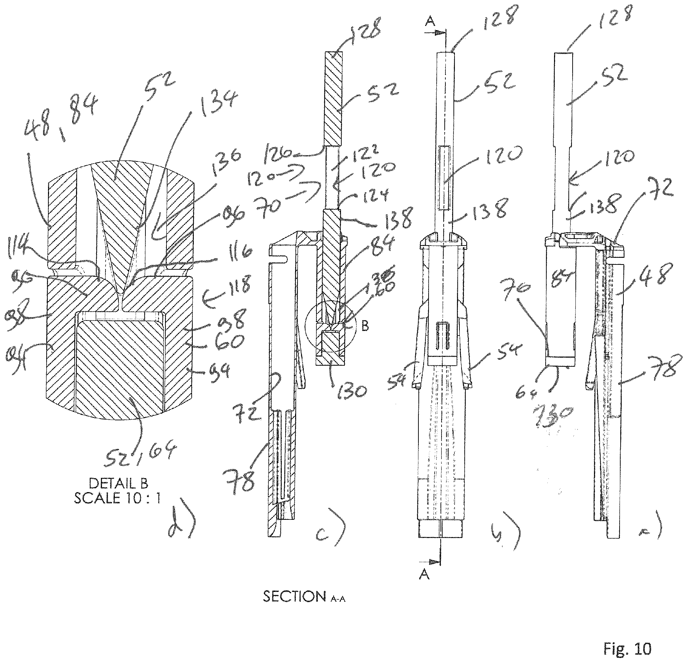

The first limb 84 is configured as a piston rod receiver 84 configured to receive and hold the piston rod 52 (see also Figs. 10 and 13).The journal 60 holds the front end 64 of the piston rod 52 in a storage state of the autoinjector.

The first limb 84 comprises a front end 76 through which the piston rod 52 extends and relative to which the piston rod 52 can move during the mixing process and the dispensing process.

The journal 60 forms a first clip component 118 of the drive chassis 48 that is configured to engage the piston rod 52.

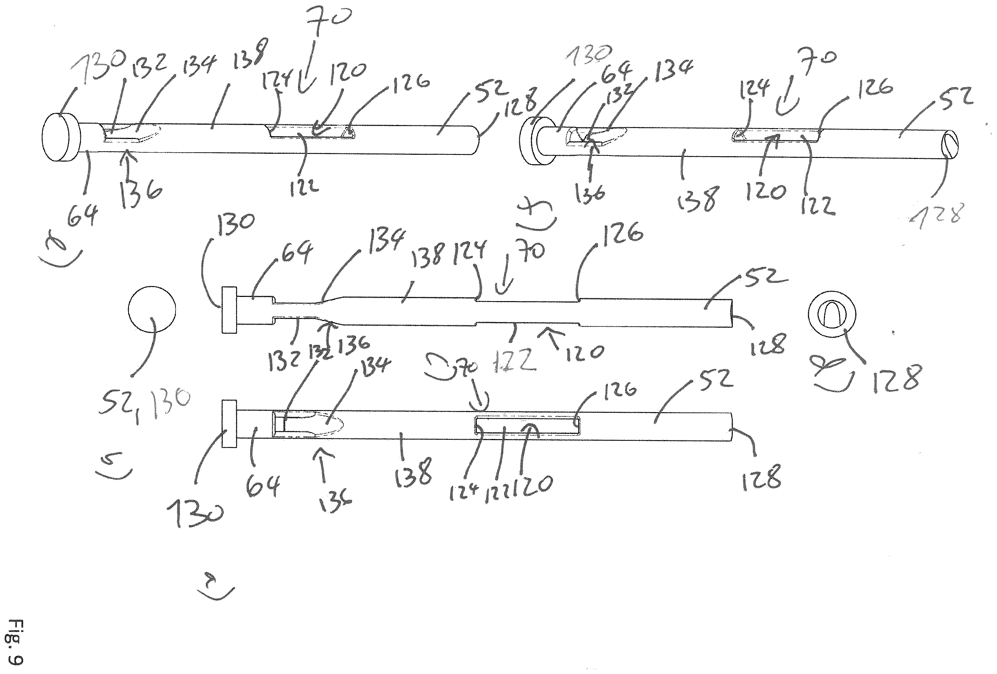

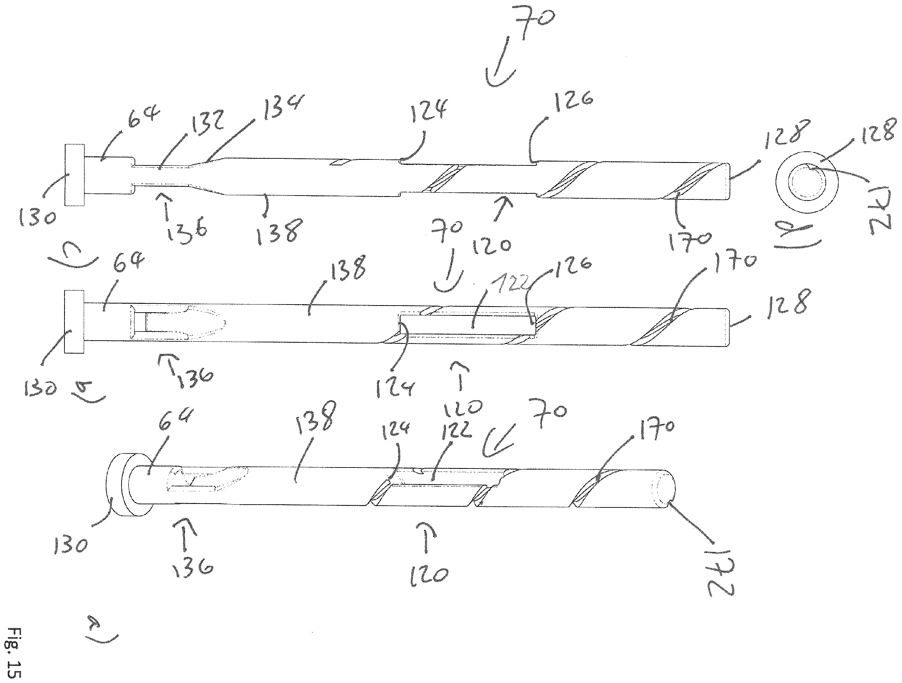

The front end 64 of the piston rod 52 is initially held relative to the front end 76 of the first limb 84 via the journal 60, i.e. the first clip component 118, in the storage state of the auto-injector 10. The front end 64 of the piston rod 52 comprises an engagement portion 136 (see e.g. Figs. 9 and 15) that is engaged by the first clip component 118 in the storage state of the autoinjector 10.

The journal 60 comprises two flexible arms 94 arranged projecting from within a window 82 of the first limb 84. During the mixing process the drive chassis 48 is axially held and the piston rod 52 moves axially in the proximal direction. This brings about a transverse deflection of the journal 60 in order to let the piston rod 52 move axially forward. In this connection it should be noted that the journal 60 is composed of flexible arms.

After a pre-determined length of travel of the piston rod 52, the journal 60 is configured to engage a second clip component 120 arranged at the middle of the piston rod 52 between the front and back ends 64 and 128 of the piston rod 52.

The pre-determined length of travel corresponds to a distance between the engagement portion 136 and the second clip component 120 of the piston rod 52.

This length can be selected in the range of 30 to 70% of a length of the piston rod 52, preferably in the range of 40 to 60% of a length of the piston rod 52.

Each of the two flexible arms 94 is at least essentially L-Shaped, with a first limb 96 extending in parallel to the first limb 84 of the drive chassis and the second limb 98 extending at least essentially transverse to the first limb 96.

The second limb 98 has a curved distal surface 114 at an end 116 of the second limb 96. This may be provided in order to aid the relative movement between the piston rod 52 and the drive chassis 48.

Figs. 9a to 9f show various views of the piston rod 52. A stopper 130 is arranged at the front end 64 that is arranged opposite to the rear end 128 of the piston rod 52. In use of the autoinjector 10, the stopper 130 of the piston rod 52 is configured to engage the distal stopper 24 of the dual chamber cartridge 11.

The second clip component 120 is arranged at approximately the middle of the piston rod 52.

The second clip component 120 comprises a first portion of reduced diameter 122, i.e. a recess or a slot, a proximal abutment 124 and a distal abutment 126.

A length of the first portion of reduced diameter 122 between the proximal abutment and the distal abutment corresponds to a length of overtravel the drive chassis 48 can travel relative to the piston rod 52 on dispensing medicament from the autoinjector 10.

The front end 64 of the piston rod 52 further comprises an engagement portion 136 that is engaged by the journal 60 respectively the first clip component 118 during a storage of the autoinjector 10.

The engagement portion 136 comprises a second portion of reduced diameter 132 and a tapered section 134.

A cylindrical portion 138 is formed between the engagement portion 136 and the second clip component 120.

Figs. 10a to d show detailed views of the interaction between the drive chassis 48 and the piston rod 52 in the storage state of the autoinjector 10.

In the storage state of the autoinjector 10, the piston rod 52 projects distally from the drive chassis 48 with more than 50% of a length of the piston rod 52 projecting distally from the drive chassis.

The stopper 130 is arranged at the front end 76 of the drive chassis 48. The journal 60 of the drive chassis 48 engages the engagement portion 136 of the piston rod 52 (see Fig. 10d).

The arms 94 of the journal 60 are arranged at the tapered section 134, especially at the smallest diameter part of the tapered section 134 in the storage state.

As will be explained in the following, the piston rod 52 can be entrained by the sleeve 62 on activation of the autoinjector 10, i.e. on carrying out a reconstitution process. When the sleeve 62 moves the piston rod 52 proximally, the drive chassis 48 is fixed in position in the outer body 46.

The proximal movement of the piston rod 52 through the journal 60 of the spring chassis 48 causes the arms 94 of the journal 60 to deflect transversely outwardly as the diameter of the tapered section 134 increases towards the cylindrical section 138, before the diameter of the piston rod 52 reduces again at the second clip component 120, where the arms 94 so to say snap into position at the first portion of reduced diameter 122 at the second clip component 120.

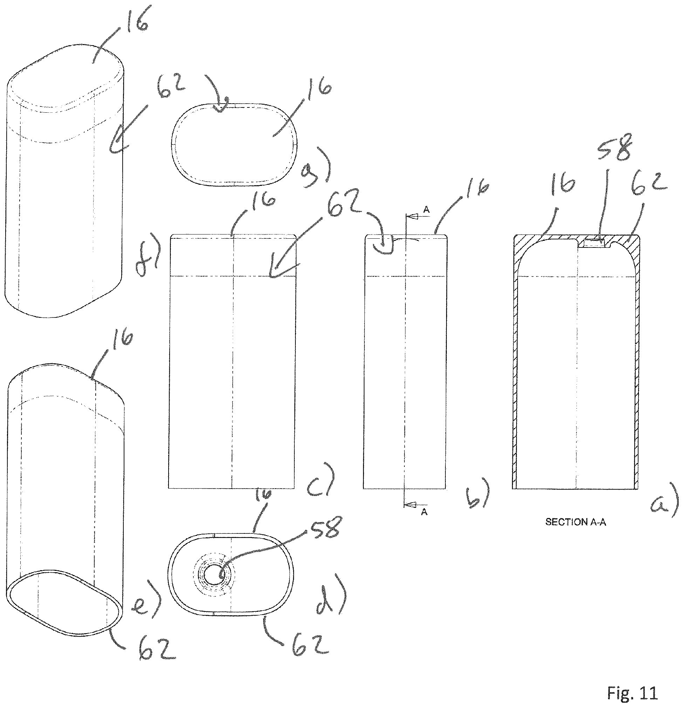

Figs. 11a to g show various views of the sleeve 62. The sleeve 62 is configured to cover the outer body 46. The sleeve 62 comprises the piston rod holder 58. The piston rod holder 58 is configured to releasably hold the piston rod 52.

This means that and the piston rod holder 58 simply constrains the piston rod 52 radially so that it can move proximally relative to the sleeve 62 in order to dispense medicament from the dual chamber cartridge 11 during the dispensing operation.

In use of the autoinjector during the activation process, the sleeve 62 is moved proximally to move the piston rod 52 relative to the outer body 46 in order to move the stoppers 22, 24 of the dual chamber cartridge 11 for the reconstitution process.

A length of a pre-defined travel of the piston rod 52 corresponds to a length of travel of the sleeve 62 relative to the outer body 46 and is limited by the distal clearancebetween the sleeve 62 and the outer body 46.

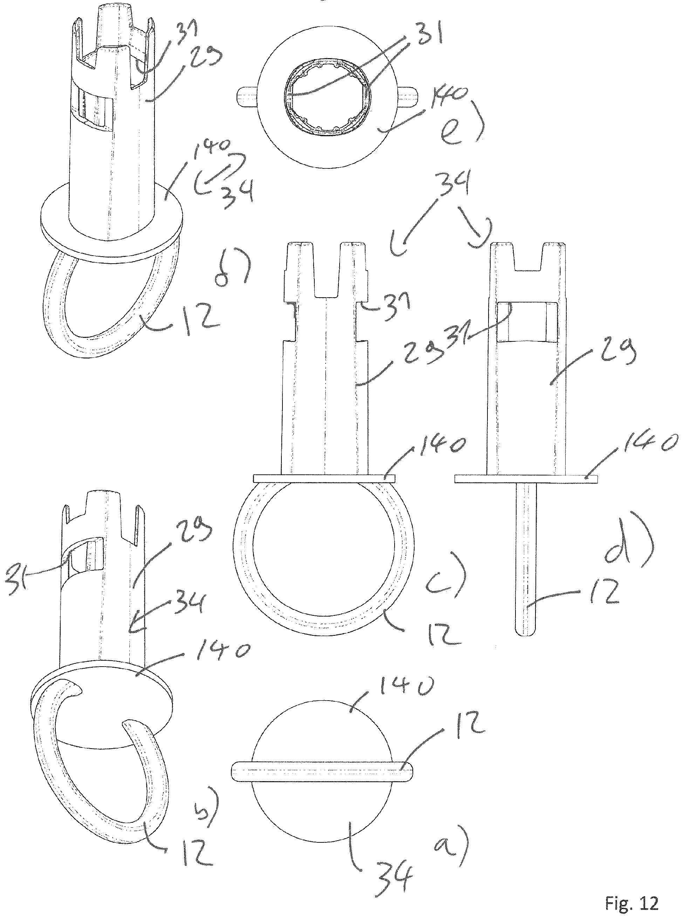

Figs. 12a to 12b show detailed views of the inner cap 32. The inner cap 34 is configured to either directly or indirectly engage the cannula shield 28.

A removal of the inner cap 34 permits a movement of air out of the dual chamber cartridge 11 and thereby facilitates the reconstitution process on activation of the autoinjector 10.

In order to remove the cannula shield 28, the inner cap 34 comprises a cannula shield holder 29 (see also Fig. 1a) at an end of the inner cap 34 disposed opposite to a base 140 of the inner cap 34.

In order to grip the cannula shield 28, also known as a needle shield 28, the inner cap 34 comprises inwardly facing projections 31 that engage the cannula shield 28. These inwardly facing projections 31 engage the cannula shield 28 in such a manner that the cannula shield is removed from the pre-filled dual chamber cartridge 11 on removal of the inner cap 34 from the autoinjector 10.

In the embodiment shown in the present application the inwardly facing projections 31 are integrally formed with the cannula shield holder 29 of the inner cap 34.

Alternatively the inwardly facing projections 31 are formed by a metal insert 30 (see e.g. Fig. 1 a) received within the cannula shield holder 29 of the inner cap 34. The metal insert 30 is fixed within the inner cap via barbs or the like (not shown) that engage the inner cap 34.

Figs. 13a to g show various views of the outer body 46, the piston rod 52, the drive chassis 48 and the sleeve 62 in the storage state, see e.g. Fig. 13a, 13b and 13c, during the medicament mixing process, see Fig. 13d, at the start or ready to start the dispensing process Fig. 13e and at the end of the dispensing process, see Fig. 13f.

Fig. 13b shows a side view of the outer body 46, and the sleeve 62. The window 90 is visible together with the projections 88 of the trigger arms 54 that engage the outer body 46 in the storage state.

Also visible are a window 144 and a clip portion 142 at the proximal end 14 of the autoinjector 10. The inner body 44 is clipped into place and thereby fixed to the outer body 46 via the clip portion 142. For this purpose the inner body 44 comprises a snap portion (not shown) that snap fits into the clip portion 142.

Regardless of the state of use of the autoinjector 10, the outer body 46 and the inner body 44 are fixed in position and do not move relative to one another.

A status of the dual chamber cartridge 11 can be viewed via the window 144. This is because the inner body 44 is beneficially formed transparent or semi transparent so a user of the autoinjector 10 can track where a position of the proximal and distal stoppers 22, 24 are in relation to the window 144 indicating whether a mixing and/or a dispensing has taken place.

Fig. 13c shows the autoinjector 10 in the storage state, i.e. in a state where the piston rod 52 is in its most distal position. In this position the drive spring 50 is received in the spring reception space 72 and is held between the outer body 46 and the base 86 of the spring reception space 72. In order to reliably hold the spring at the outer body 46, this comprises an inwardly facing projection 146 formed as a drive spring holder.

The position of the piston rod 52 and the drive chassis 48 in the storage state corresponds to that shown in Fig. 10.

The drive chassis 48 is fixed in position and does not move relative to the outer body 46, the sleeve 62, the piston rod 52 in the storage state.

During activation of the autoinjector 10, the drive chassis 38 is likewise held fixed in position relative to the outer body 46. In contrast to this the sleeve 62 and the piston rod 52 are moveable relative to the drive chassis 48 and the outer body 46.

The sleeve 62 is moved proximally thereby entraining the piston rod 53, this causes the tapered section 134 of the piston rod 52 to deflect the arms 94 of the journal 60 so that this can slide over the cylindrical portion 138. Following which the arms 94 snap into place at the second clip component 120 as is shown in Fig. 13d.

The piston rod 52 continues to travel proximally having reached the second clip component 120 until sleeve 62 contacts an end face 148 of the outer body 46 which happens at the same time as the journal 60 approaches the distal abutment surface 126. When the sleeve 62 contact the end face 148, the reconstitution or mixing process is completed. This state is indicated in Fig. 13e.

As the reconstitution or mixing process is completed, the piston rod 52 is released from the piston rod holder 58 of the sleeve 62 in order to enable a movement of the piston rod 52 relative to the sleeve 62 and the outer body 46 during the dispensing process, see e.g. Fig. 13f.

Once a user of the autoinjector 10 applies the cannula guard 38 to a skin of a patient (not shown), see e.g. the state shown in Figs. 1e to f, the distal movement of the needle guard releases the drive chassis 48 from its engagement with the outer body 46.

The release of the drive chassis 48 from engagement with the outer body 46 enables the force of the drive spring 50 to drive the drive chassis 48 proximally. During an initial phase of travel of the drive chassis 48, the piston rod 52 may not be moved relative thereto.

During this phase of travel of the drive chassis 48, the journal moves over the first portion of reduced diameter 122 towards the proximal abutment surface 124. Once the journal 60 of the drive chassis 48 contacts the proximal abutment surface 124 the piston rod 52 is moved proximally with respect to the outer body 46, the inner body 44 and the sleeve 62 in order to dispense a medicament from the dual chamber cartridge 11.

This means that the piston rod 52, once detached from piston rod holder 58, is entrained by the drive chassis 48 via the force of drive spring 50. In this connection it should be noted that the piston rod 52 may only be entrainable once it is released from the piston rod holder 58.

The distance the drive chassis 48 travels along the first portion of reduced diameter 122 is a distance of over travel of the drive chassis 48 relative to the inner body 44 and the outer body 46.

This distance of over travel can be required in order that the total travel is sufficient to bring about a lock-out of the autoinjector 10 if provided. Such a lock out can be provided between the drive chassis 48, needle guard 56 and the inner body 44 (see Fig. 1f and g) or outer body 46 (not shown) and/or to compensate for an increase in length of the autoinjector due to the dual chamber cartridge 11.

A Needle Guard 56 lockout mechanism of the autoinjector may require a certain travel of the spring chassis 48 during dispense (>26mm in the embodiment shown). This may be greater than the stopper travel of the proximal and distal stoppers 22, 24 required to dispense the volume of mixed medicament required.

To enable the spring chassis 48 travel to exceed the travel required in the stoppers 22, 24, the piston rod 52 may over travel past its clip abutment with the spring chassis 48, i.e. the first clip component 60 may travel over the first portion of reduced diameter 122. In this way, when the autoinjector 10 is triggered, the spring chassis 48 can move forward a defined amount before picking up the piston rod 52 via the proximal clip abutment surface 124 and moving it to dispense a dose. This can allow the spring chassis 48 to complete its minimum required displacement, even for low fill variants.

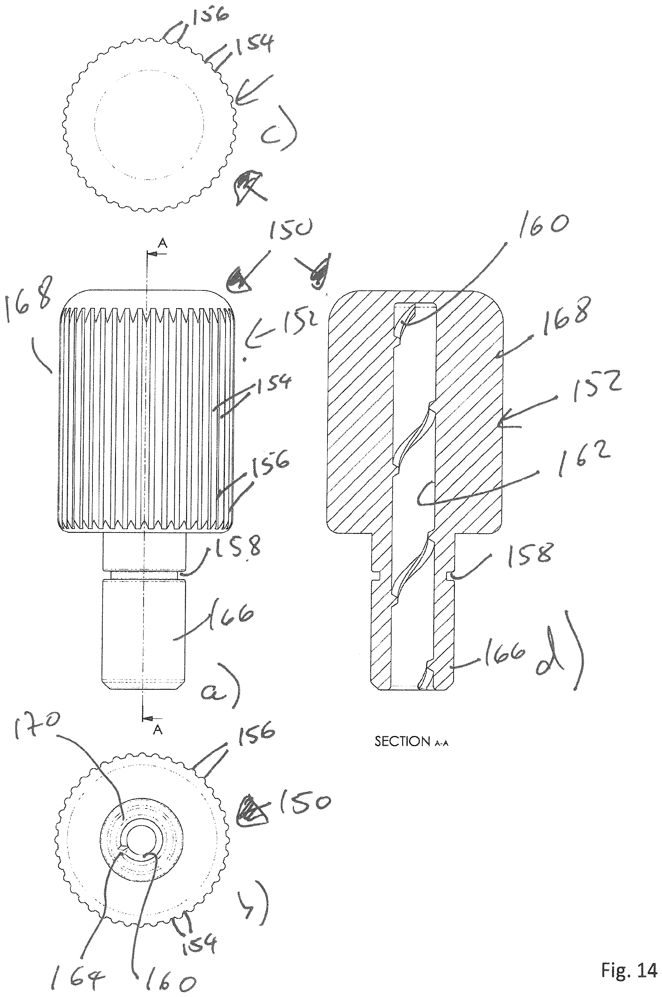

Fig. 14a to d show various views of a dial 150. The dial comprises a gripping surface 152. The gripping surface 152 in the example shown comprises a plurality of grooves and projections 154, 156 extending in parallel to one another.

The dial 150 is formed at a further kind of autoinjector 10 and is used to move the piston rod 52 proximally in order to carry out a reconstitution or mixing process. The dial 150 can be provided as an alternative to the sleeve 62 shown in the foregoing.

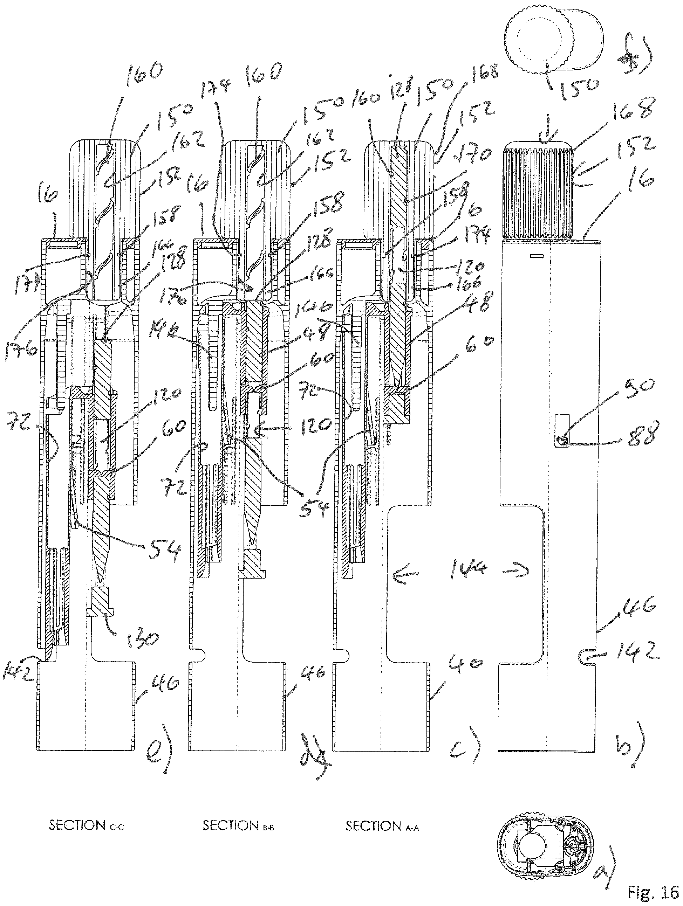

The gripping surface 152 ensures that a user can reliably grip the dial 150 in order to rotate this. The dial 150 is axially fixed to the outer body 46 (see also Fig. 16) via an annular recess 158 in such a manner that it can be rotated relative to the outer body 46.

The annular recess 158 is provided on a portion of smaller diameter 166 whereas the gripping surface 152 is provided on a portion of larger diameter 168 of the dial 150.

The dial 152 comprises an inner thread 160 within a piston rod receiving space 162 formed within the dial 152 as indicated in Fig. 14d. By means of this inner thread 160, the piston rod 52 can be rotated and moved proximally during a reconstitution process.

The inner thread 160 extends transversely to the annular recess 158 in such a way that a rotation of the dial 150 about the piston rod receiving space 162 causes a proximal movement of the piston rod 52.

Fig. 14 b shows a view from below the dial with a thread start 164 of the inner thread 160 being provided at a proximal end 170 of the dial 150.

Rather than providing an annular recess 158, the portion of smaller diameter 166 could be provided with an outer thread and could be entrained together with the piston rod 52 for a proximal movement, in this case the piston rod would simply be held in the piston rod receiving space 162, either by splines or a different form of holding connection.

Other design options are that the piston rod 52 is threaded to outer body and splined to the dial or is threaded to the dial and splined to the outer body (both not shown).

Figs. 15a to d show views of a piston rod 52 formed complementary to the dial 150 of Fig. 14. The function of the features that are equivalent to those shown in connection with Fig. 9 are the same as those discussed in connection with Fig. 9.

This means the piston rod 52 of Fig. 15 has the first clip component 120 such that the piston rod 52 of Fig. 14 can be entrained by the drive chassis 48 in the same way as the drive chassis 48 of Fig. 8. In fact the same design of drive chassis 48 can be used for both designs of piston rod 52.

The difference lies in an outer thread 170 that is provided at the piston rod 52 of Fig. 15. The outer thread extends from a thread end 172 at the distal end of the piston rod 52 through the middle portion 70 of the piston rod up to and into the cylindrical portion 136.

A length of the outer thread 170 is selected such that it ensures a proximal travel of the piston rod 52 during reconstitution or mixing and the outer thread 170 is configured such that it becomes detached from the dial at the end of the reconstitution or mixing process via the end of thread 172.

Fig. 16a to f show views of the second kind of autoinjector 10 similar to the views shown in Fig. 13a to g. The difference lies in the provision of the dial 150 rather than the sleeve 62 and the fact that a length of the outer body 46 is increased with respect to that shown in Fig. 13.

The portion of smaller diameter 156 having the annular recess 158 of the dial 150 is received within a channel 176 of the outer body 46.

The outer body 46 comprises an annular projection 174 shaped complementary to the annular recess 158 of the dial 150 within the channel 176. The dial 150 is thereby axially fixed to the outer body 46 and rotatable relative thereto.

On activating the autoinjector 10 a user can rotate the dial 150 in order to move the piston rod 52 relative to the outer body 46 and the drive chassis 48. Once the outer thread 170 of the piston rod 52 detaches from the inner thread 160 of the dial 150 (see e.g. Fig. 16d), a dispensing process can be started and the drive chassis 48 initially over-travels over the first portion of reduced diameter 122 of the piston rod prior to abutting the proximal abutment surface 124 for entraining the piston rod 52 proximally, as indicated in Fig. 16e.

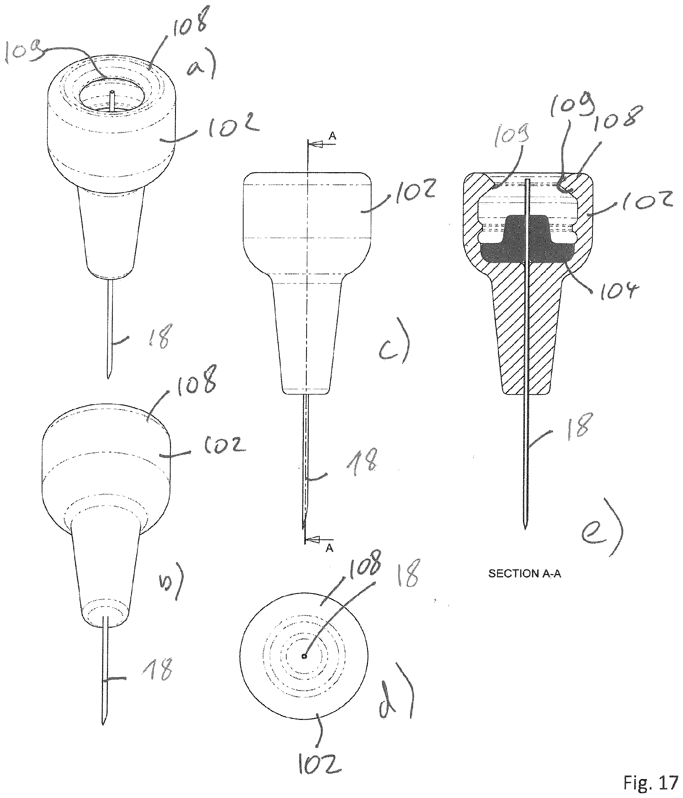

Fig. 17a to e show various views of a cap 102 of the cap and seal assembly 100 shown in Fig. 2 and 3. The cap 102 comprising the cannula 18 is configured to be attached to the opening 106 of the dual chamber cartridge 11. The seal 104 is provided in order to prevent medicament from escaping through the opening by means other than the cannula 18.

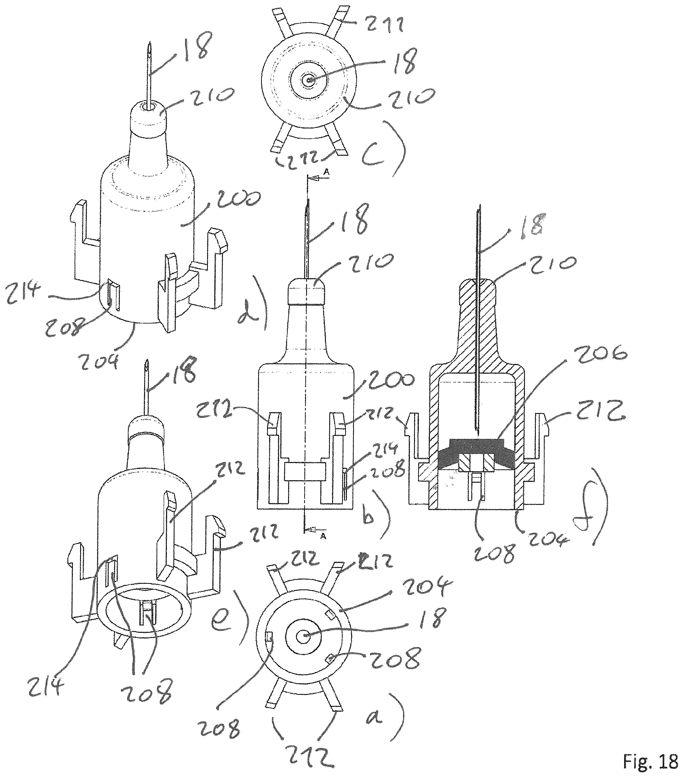

Fig. 18a to f show various views of a second kind of carriage 200. The difference to the carriage shown in Figs. 4 to 6 lies in the type of detents 208. Rather than projecting from the distal end 204, these are formed at the distal end 204 and are arranged in windows 214 of the carriage 200.

Also visible are wings 212 via which the carriage 200 is snap fit into the inner body 44 of the autoinjector 10. The carriage 200 is fixedly held in the autoinjector 10, so that on activating the autoinjector 10, the movement of the sleeve 62 relative to the inner body 44, the dual chamber cartridge 11 is moved into engagement with the carriage 200 such that the cannula 18 pierces the seal 206 in order to facilitate the reconstitution process, i.e. the mixing process and to enable a dispensing from the dual chamber cartridge 11 after the mixing process has taken place.

### List of reference numerals:

- 10: autoinjector
- 11: dual chamber cartridge
- 12: handle
- 14: proximal end
- 16: distal end
- 18: cannula
- 20: pre-filled syringe
- 22: proximal stopper
- 23: first chamber
- 24: distal stopper
- 25: second chamber
- 26: bypass passage
- 28: removable cannula shield
- 29: cannula shield holder
- 30: metal pressing
- 31: projections
- 32: cap
- 34: inner cap
- 36: aperture
- 38: cannula guard
- 40: housing
- 42: lock-out spring
- 44: inner body
- 46: outer body
- 48: drive chassis
- 50: drive spring
- 52: piston rod
- 54: trigger arms
- 56: cannula guard plunger
- 58: piston rod holder
- 60: journal of spring chassis/first clip component
- 62: sleeve
- 64: front end of piston rod
- 66: end of dose click arm
- 68: end of dose click ramp
- 70: middle portion of 52
- 72: spring reception space
- 74: piston rod reception space
- 76: front end of 74
- 78: spring receiver/second limb of 48
- 80: guide/groove
- 82: window
- 84: piston rod receiver/first limb of 48
- 86: base
- 88: projection at 54
- 90: structure/window
- 92: spline
- 94: arms of 60
- 96: first limb of 60
- 98: second limb of 60
- 100: cap and seal assembly
- 102: cap
- 104: seal
- 106: opening
- 108: first flange arrangement
- 109: undercut
- 110: second flange arrangement
- 111: web
- 112: rubber septum
- 114: curved distal surface
- 116: end of 98
- 118: first clip component
- 120: second clip component
- 122: first portion of reduced diameter
- 124: proximal abutment surface
- 126: distal abutment surface
- 128: rear end of 52
- 130: stopper
- 132: second portion of reduced diameter
- 134: tapered section
- 136: engagement portion
- 138: cylindrical portion
- 140: base
- 142: clip portion
- 144: window
- 146: spring holder
- 148: end face of 46
- 150: dial
- 152: gripping portion
- 154: projection
- 156: groove
- 158: annular recess
- 160: inner thread
- 162: piston rod receiving space
- 164: thread start
- 166: portion of small diameter of 150
- 168: portion of larger diameter of 150
- 170: outer thread
- 172: thread end
- 174: annular projection
- 176: channel
- 200: cannula carriage
- 201: cannula carriage reception space
- 202: rubber septum
- 204: distal end
- 206: seal
- 208: detent
- 210: proximal end
- 212: wings
- 214: window

Enumerated embodiments:
   1. An autoinjector comprising a dual chamber component, a body, a spring chassis and a piston rod, wherein the piston rod is configured to be moved relative to the body and through the spring chassis, wherein the spring chassis has a first clip component and the piston rod has a second clip component configured complementary to the first clip component, wherein the first and second clip components are configured to engage once the piston rod has travelled a pre-determined distance through the spring chassis.
   2. The autoinjector of embodiment 1, wherein, following engagement of the first and second clip components, the drive chassis is configured to entrain the piston rod for a common axial movement during the dispensing operation.
   3. The autoinjector of embodiment 1 or embodiment 2, further comprising a sleeve in which a part of the body is received.
   4. The autoinjector of embodiment 3, wherein the piston rod is in axial contact and/or held at the sleeve.
   5. The autoinjector of embodiment 3 or embodiment 4, wherein the piston rod is releasably held at the sleeve.
   6. The autoinjector of one of embodiments 3 to 5, wherein the piston rod is configured to be disengaged from the sleeve once the first and second clip components have engaged.
   7. The autoinjector of one of embodiments 3 to 6, wherein the sleeve is configured to move the piston rod on activation of the autoinjector.
   8. The autoinjector of embodiment 7, wherein sleeve is configured to move the piston rod relative to the spring chassis during the activation of the autoinjector.
   9. The autoinjector of one of embodiments 7 to 8, wherein the piston rod is configured to move relative to the first clip component of the spring chassis during the activation of the autoinjector.
   10. The autoinjector of one of embodiments 1 to 9, wherein the piston rod comprises a tapered section adjacent to the second clip component.
   11. The autoinjector according to embodiment 10, wherein the tapered section increases in size towards the second clip component.
   12. The autoinjector according to embodiment 10 or embodiment 11, wherein the first clip component is configured to move over the tapered section.
   13. The autoinjector of one of embodiments 3 to 12, wherein the body is slideably mounted in the sleeve.
   14. The autoinjector of one of embodiments 1 to 13, further comprising a dial.
   15. The autoinjector of embodiment 14, wherein the dial is rotatable but axially fixed to the body.
   16. The autoinjector of embodiment 14 or embodiment 15, wherein the piston rod is releasably held at the dial.
   17. The autoinjector of one of embodiments 14 to 16, wherein the piston rod is configured to be disengaged from the dial once the autoinjector has been activated.
   18. The autoinjector of embodiment 17, wherein the piston rod is configured to be disengaged from the dial before the first and second clip components have engaged.
   19. The autoinjector of one of embodiments 14 to 18, wherein the dial is configured to move the piston on activation of the autoinjector.
   20. The autoinjector of one of embodiments 14 to 19, wherein the dial is configured to rotate in order to drive the piston rod axially for the pre-determined distance.
   21. The autoinjector of embodiment 20, wherein the piston rod is splined to a housing component and is only axially driven.
   22. The autoinjector of embodiment 20, wherein the piston rod is splined to the dial and is axially and rotationally driven.
   23. The autoinjector of one of embodiments 1 to 22, wherein the drive spring chassis is an at least generally U-shaped drive spring chassis having first and second limbs, with the piston rod being guided in the first limb.
   24. The autoinjector of one of embodiments 1 to 23, wherein the drive spring chassis is slideably mounted in the body.
   25. The autoinjector of one of embodiments 1 to 24, further comprising a needle guard, wherein a distal movement of the needle guard releases the spring chassis from the storage position to bring about, i.e. enable, a proximal movement of the spring chassis in the body for dispensing a medicament stored in said autoinjector in a dispensing operation.
   26. The autoinjector in accordance with one of embodiments 1 to 25, wherein the dual chamber component comprises first and second chambers for first and second elements of a medicament that can each be stored in the respective first and second chambers of the dual chamber component, with the first and second chambers being separated from one another by a proximal stopper in a storage state of the autoinjector.
   27. The autoinjector in accordance with embodiment 26, wherein the pre-determined distance of travel of the piston rod is reached once a distal stopper of the dual chamber component has reached the proximal stopper.
   28. The autoinjector of embodiment 25 or embodiment 26, further comprising said two elements of medicament and said second element stored in the chamber between the proximal and distal stopper, wherein a mixing process of the autoinjector is completed once the second element has been mostly urged into the first chamber by a proximal movement of the distal stopper.
   29. The autoinjector of one of embodiments 25 to 28, wherein said dispensing operation only takes place after the first and second clip components have engaged.
   30. The autoinjector of one of embodiments 1 to 29, wherein the spring chassis is fixed to the body during a medicament mixing process.
   31. The autoinjector of one of embodiments 1 to 30, wherein the piston rod moves relative to the body during a medicament mixing process.
   32. The autoinjector of one of embodiments 1 to 31, wherein the piston rod and the spring chassis move during a medicament dispensing operation.
   33. The autoinjector of one of embodiments 1 to 32, wherein the second clip component of the piston rod comprises a portion of reduced diameter with respect to directly adjacent sections of the piston rod.
   34. The autoinjector of one of embodiments 1 to 33, wherein the second clip component comprises a proximal abutment arranged directly adjacent to the portion of reduced diameter.
   35. The autoinjector of embodiment 34, wherein, during the dispensing operation, a movement of the piston rod only takes place after the first clip component engages the proximal abutment of the second clip component.
   36. The autoinjector of one of embodiments 1 to 35, wherein, during the dispensing operation, the spring chassis initially moves relative to the body and to the piston rod prior to engagement of the first clip component with the proximal abutment.
   37. The autoinjector of one of embodiments 1 to 36, wherein the second clip component comprises a distal abutment.
   38. The autoinjector of one of embodiments 34 to 36 and embodiment 37, wherein the distal abutment and the proximal abutment are arranged at oppositely disposed ends of the portion of reduced diameter.
   39. The autoinjector of one of embodiments 33 to 38, wherein the first clip component can move relative to the portion of reduced diameter.
   40. A method of reconstituting a medicament stored in an autoinjector, the method comprising the steps of:
      moving a piston rod of an autoinjector through a drive chassis of said autoinjector to move a distal stopper of a dual chamber component proximally, e.g. into contact with a proximal stopper of the dual chamber component.
41. An autoinjector comprising a dual chamber cartridge (11) comprising two elements of a medicament (M, M') that are each stored in a separate chamber (23, 24) of the dual chamber cartridge (11) and the dual chamber cartridge (11) comprising a bypass passage (26) and the autoinjector (10) further comprising at least one security mechanism configured to secure the autoinjector against premature mixing of the elements and dispensing of said mixed elements (M, M').
42. An autoinjector, in particular according to embodiment 41, comprising a dual chamber cartridge (11) comprising two elements of a medicament (M, M') that are each stored in a separate chamber (23, 24) of the dual chamber cartridge (11) and the dual chamber cartridge (11) comprising a bypass passage (26), the dual chamber cartridge (11) comprising a syringe cap and seal assembly (100) covering and fixed to an outlet from the dual chamber cartridge (11).
43. The autoinjector of embodiment 42, wherein the cap and seal assembly (100) comprises a cap (102) and one or more seals (104) surrounded by the cap (102).
44. The autoinjector of embodiment 43, wherein the dual chamber cartridge (11) has an opening (106) which is covered by the cap (102) and sealed off by one of said one or more seals (104) of the cap and seal assembly (100).
45. The autoinjector of one of embodiments 42 to 44, wherein the cap and seal assembly (100) comprises a first flange arrangement (108) and the dual chamber cartridge (11) comprises a second flange arrangement (110) shaped complementary to the first flange arrangement (108) and being engageable by the first flange arrangement (108).
46. The autoinjector of embodiment 45, wherein the first flange arrangement (108) surrounds said second flange arrangement (110).
47. The autoinjector of embodiment 45 or embodiment 46, wherein the first flange arrangement (108) is snap-fit into place at the second flange arrangement (110).
48. The autoinjector of one of embodiments 42 to 47, further comprising a cannula (18) held in place at the dual chamber cartridge (11) by the cap and seal assembly (100).
49. The autoinjector of embodiment 8 and one of embodiments 43 to 47, wherein the cannula (18) is held in place at the dual chamber cartridge (11) by one of said one or more seals (104) of the cap and seal assembly (100).
50. The autoinjector of one of embodiments 42 to 49, wherein the cap and seal assembly (100) further comprises a cannula shield (28) covering the cap and seal assembly (100).
51. The autoinjector of one of embodiments 42 to 50, wherein the dual chamber cartridge (11) further comprises a proximal (22) and a distal stopper (24).
52. The autoinjector of one of embodiments 42 to 51, wherein the dual chamber cartridge (11) comprises a first chamber (23) more proximally arranged than a second chamber (25), wherein the first chamber (23) is filled with a solid material (M) and the second chamber (25) is filled with a liquid material (M').
53. The autoinjector of embodiments 51 and embodiment 52, wherein the first chamber (23) is separated from the second chamber (25) by the proximal stopper (22).
54. The autoinjector of one of embodiments 51 to 53, wherein the proximal stopper (22) is arranged distally of the bypass passage (26) in a storage state of the auto-injector (10).
55. The autoinjector of one of embodiments 51 to 54, wherein the proximal stopper (22) is arranged proximally of the bypass passage (26) after a dispensing operation has taken place.
56. The autoinjector of one of embodiments 42 to 55, further comprising a piston rod (52) configured to act on said distal stopper (24) and said proximal stopper (22) and to thereby move the proximal stopper (22) such that it is arranged at the bypass passage (26) for urging, i.e. allowing, the material into the proximal chamber (23) past the proximal stopper (22) via the bypass passage (26) during a mixing process.
57. The autoinjector of embodiment 56, wherein the piston rod (52) can be moved further for dispensing the medicament via the cap and seal assembly (100).
58. The autoinjector of one of embodiments 44 to 57, wherein the one of said one or more seals (104) of the cap and seal assembly (100) sealing off the opening (106) covers said opening (106).
59. The autoinjector of one of embodiments 42 to 58, further comprising a cannula shield (28) such as a rigid cannula shield (28) covering a cannula (18) of the pre-filled syringe.
60. A method of mixing a medicament in an autoinjector (10), the autoinjector (10) comprising a dual chamber cartridge (11) comprising first and second elements (M, M') of a medicament, with the first and second elements (M, M') each being stored in a separate chamber (23, 25) of the dual chamber cartridge (11), the dual chamber cartridge (11) comprising a cap and seal assembly (100) as well as a piston rod (52), the method comprising the steps of:
   - moving the piston rod (52) proximally towards a distal stopper (24) and thereby moving said distal stopper (24) towards a proximal stopper (22) and reducing the distance between the distal stopper (24) and said proximal stopper (22) to thereby urge a first element (M) of the medicament from a first chamber (23) into a bypass passage (26) to bypass the proximal stopper (25) such that the first element (M) comes into contact with the second element (M') in the first chamber (25) arranged between the cap and seal assembly (100) and the proximal stopper (22).
61. An autoinjector, in particular according to embodiment 41, comprising a dual chamber cartridge (11) comprising two elements (M, M') of a medicament that are each stored in a separate chamber (23, 25) of the dual chamber cartridge (11), the dual chamber cartridge (11) comprising a bypass passage (26) and a rubber septum (202) and a cannula shield (28), the autoinjector (10) further comprising a cannula carriage (200) having a cannula (18) with the cannula carriage (200) being in contact with the dual chamber cartridge (11) in a storage state of the autoinjector (10) and the dual chamber cartridge (11) being moveable towards the cannula carriage (200) on activation of the autoinjector (10) in order to penetrate the septum (202) with the cannula (18).
62. The autoinjector of embodiment 61, wherein a distal end (204) of the cannula carriage (200) is sealed off by a seal (206).
63. The autoinjector of embodiment 62, wherein the seal (206) is moveably arranged in a distal cannula carriage reception space (201) relative to the cannula (18).
64. The autoinjector of one of embodiments 61 to 63, wherein the cannula carriage (200) comprises one or more detents (208) at a distal end (204) thereof.
65. The autoinjector of embodiment 64, wherein the cannula carriage (200) is in contact with the dual chamber cartridge (11) via the one or more detents (208) in the storage state of the autoinjector (10).
66. The autoinjector of embodiment 64 or embodiment 65, wherein, on activation of the autoinjector (10), the dual chamber cartridge (11) is configured to overcome said one or more detents (208) such that the cannula (18) pierces the septum (202).
67. The autoinjector of one of embodiments 62 to 66, wherein a movement of the dual chamber cartridge (11) towards the cannula carriage (200) moves said seal (206) towards the cannula (18) to pierce said seal (206) on activation of the autoinjector (10).
68. The autoinjector of embodiment 64 and one of embodiments 62 to 66, wherein the movement of the dual chamber cartridge (11) towards the cannula carriage (200) moves said seal (206) towards the cannula (18) to first pierce said seal (206) and then secondly pierce said septum (202).
69. The autoinjector of one of embodiments 61 to 68, further comprising a distal stopper (24) arranged in said dual chamber cartridge (11), and a piston rod (52), wherein, on activation of said autoinjector (10), a movement of said piston rod (52) towards the distal stopper (24) brings about a movement of said dual chamber cartridge (11) towards said cannula carriage (200).
70. The autoinjector of one of embodiments 64 to 67 and embodiment 69, wherein, once said septum (202) is pierced, movement of said distal stopper (24) within the dual chamber cartridge (11) is facilitated.
71. The autoinjector of one of embodiments 61 to 69, wherein the dual chamber cartridge (11) comprises a proximal (22) and a distal stopper (24) and a bypass passage (26).
72. The autoinjector of one of embodiments 61 to 70, wherein the dual chamber cartridge (11) comprises a first chamber (23) more proximally arranged than a second chamber (25), wherein the first chamber (23) is filled with a solid material (M) of said medicament and the second chamber (25) is filled with a liquid material (M') of said medicament.
73. The autoinjector of embodiments 71 and embodiment 72, wherein the first chamber (23) is separated from the second chamber (25) by the proximal stopper (22).
74. The autoinjector of one of embodiments 71 to 73, further comprising a piston rod (52) configured to act on said distal stopper (24) and said proximal stopper (22) and to thereby move the proximal stopper (22) such that it is arranged at the bypass passage (26) for urging the liquid material (M') into the first chamber (23) past the proximal stopper (22) via the bypass passage (26).
75. A method of activating an autoinjector, the autoinjector (10) comprising a dual chamber cartridge (11) comprising first and second elements (M, M') of a medicament, with the first and second elements (M, M') each being stored in a separate chamber (23, 25) of the dual chamber cartridge (11), a cannula carriage (200) having a cannula (18) and a piston rod (52), the method comprising the steps of:
   - moving the piston rod (52) proximally towards a distal stopper (24) arranged in the dual chamber cartridge (11) and thereby moving said dual chamber cartridge (11) towards the cannula carriage (200) to pierce a septum (202) of said dual chamber cartridge (11) with said cannula (18).
76. The method of embodiment 75, further comprising the step of:
   allowing air to be expelled from said dual chamber cartridge (11) via said cannula (18) and then further moving the distal stopper (24) towards a proximal stopper (22) to thereby urge a second element (M') of the medicament from a second chamber (25) into a bypass passage (26) to bypass the proximal stopper (22) such that the second element (M') comes into contact with the first element (M) in the first chamber (23) arranged between the cannula carriage (200) and the proximal stopper (22).
77. An autoinjector, in particular according to embodiment 41, comprising a dual chamber cartridge (11) comprising two elements (M, M') of a medicament that are each stored in a separate chamber (2, 25) of the dual chamber cartridge (11) and the dual chamber cartridge (11) comprising a bypass passage (26) and an outer cap (34), an inner cap (34) and a cannula shield (28) covering a cannula (18) of the autoinjector (10), wherein, on activation of the autoinjector (10) said inner cap (34) is removable from the autoinjector (10) before said outer cap (34) is removed from said autoinjector (10).
78. The autoinjector of embodiment 77, wherein the autoinjector (10) is configured such that removal of the inner cap (34) simultaneously brings about a removal of the cannula shield (28).
79. The autoinjector of embodiment 77 or embodiment 78, wherein the inner cap (34) comprises a grip (12) projecting from the autoinjector (10).
80. The autoinjector of embodiment 79 wherein the grip (12) comprises a ring.
81. The autoinjector of one of embodiments 77 to 80, wherein removal of the inner cap (34) permits a movement of air out of the dual chamber cartridge (11).
82. The autoinjector of one of embodiments 77 to 81, wherein the inner cap (34) comprises a cannula shield holder (29) at an end of the inner cap (34) disposed opposite to a base of the inner cap (34).
83. The autoinjector of one of embodiments 77 to 82, wherein the inner cap (34) comprises inwardly facing projections (31) that engage the cannula shield (28).
84. The autoinjector of embodiment 82 and embodiment 83, wherein the inwardly facing projections (31) are integrally formed with the cannula shield holder (29) of the inner cap (34).
85. The autoinjector of embodiment 82 and embodiment 83, wherein the inwardly facing projections are formed by a metal insert (30) received within the cannula shield holder (29) of the inner cap (34).
86. The autoinjector of one of embodiments 77 to 85, wherein the outer cap (34) is configured to receive a part of a cannula shield (28) of the autoinjector (10).
87. The autoinjector of one of embodiments 77 to 86, wherein an axial movement of a cannula guard (38) of the autoinjector (10) in the direction of the dual chamber cartridge (11) brings about a release of a drive mechanism of a plunger of the dual chamber cartridge (11) for dispensing a medicament (M, M') stored in the dual chamber cartridge (11).

## Claims

1. An autoinjector comprising a dual chamber component, a body, a spring chassis and a piston rod, wherein the piston rod is configured to be moved relative to the body and through the spring chassis, wherein the spring chassis has a first clip component and the piston rod has a second clip component configured complementary to the first clip component, wherein the first and second clip components are configured to engage once the piston rod has travelled a pre-determined distance through the spring chassis.

2. The autoinjector of claim 1, wherein, following engagement of the first and second clip components, the drive chassis is configured to entrain the piston rod for a common axial movement during the dispensing operation.

3. The autoinjector of claim 1 or claim 2, further comprising a sleeve in which a part of the body is received.

4. The autoinjector of one of claims 1 to 3, wherein the piston rod comprises a tapered section directly adjacent to the second clip component.

5. The autoinjector according to claim 4, wherein the tapered section increases in size towards the second clip component.

6. The autoinjector according to claim 4 or claim 5, wherein the first clip component is configured to move over the tapered section.

7. The autoinjector of one of claims 3 to 12, wherein the body is slideably mounted in the sleeve.

8. The autoinjector of one of claims 1 to 7, further comprising a dial.

9. The autoinjector of claim 8, wherein the dial is rotatable but axially fixed to the body.

10. The autoinjector of claim 9 or claim 10, wherein the piston rod is releasably held at the dial.

11. The autoinjector of one of claims 14 to 16, wherein the piston rod is configured to be disengaged from the dial once the autoinjector has been activated.

12. The autoinjector of one of claims 1 to 1, wherein a dispensing operation only takes place after an activation of the autoinjector and further after the first and second clip components have engaged.

13. The autoinjector of one of claims 1 to 12, wherein the second clip component of the piston rod comprises a portion of reduced diameter with respect to directly adjacent sections of the piston rod.

14. The autoinjector of one of claims 1 to 13, wherein the second clip component comprises a proximal abutment arranged directly adjacent to the portion of reduced diameter.

15. The autoinjector of claim 14, wherein, during the dispensing operation, a movement of the piston rod only takes place after the first clip component engages the proximal abutment of the second clip component.

16. The autoinjector of one of claims 1 to 15, wherein, during the dispensing operation, the spring chassis initially moves relative to the body and to the piston rod prior to engagement of the first clip component with the proximal abutment.

17. The autoinjector of one of claims 13 to 16, wherein the first clip component can move relative to the portion of reduced diameter.

18. A method of reconstituting a medicament stored in an autoinjector, in particular an autoinjector according to one of the preceding claims, the method comprising the steps of:
moving a piston rod of an autoinjector through a drive chassis of said autoinjector to move a distal stopper of a dual chamber component proximally
